# EUROPEAN PATENT APPLICATION

(11) **EP 3 799 888 A1**
(43) Date of publication of application: **07.04.2021**
(21) Application number: 19200824.1
(22) Date of filing: 01.10.2019
(51) Int. Cl.: A61K 47/69, A61K 47/68, A61P 35/00

(54) **LIPOSOMES COMPRISING ANTI-LOX ANTIBODY**

(71) Applicant: Istituto Scientifico Romagnolo Per Lo Studio E La Cura Dei Tumori (I.R.S.T.) S.R.L., 47014 Meldola (FC) (IT); Houston Methodist Hospital, Houston, TX 77030 (US)
(72) Inventor: De Vita, Alessandro, 47014 Meldola (FC) (IT); Liverani, Chiara, 47014 Meldola (FC) (IT); Ibrahim, Toni, 47014 Meldola (FC) (IT); Mercatali, Laura, 47014 Meldola (FC) (IT); Tasciotti, Ennio, Houston, TX 77030 (US); Molinaro, Roberto, Houston, TX 77030 (US)
(74) Representative: Trillat, Anne-Cecile

(57) **Abstract**

The present invention relates to a liposome comprising a poly(ethylene glycol)-lipid (PEG-lipid), cholesterol and an anti-LOX antibody and relative pharmaceutical formulation, medical uses in particular for the treatment of cancer.

## Description

### TECHNICAL FIELD

The present invention relates to a liposome comprising a poly(ethylene glycol)-lipid (PEG-lipid), cholesterol and an anti-LOX antibody and relative pharmaceutical formulation and medical uses, in particular for the treatment of cancer.

### BACKGROUND ART

In the landscape of drug delivery systems, a multitude of nanovectors have been developed for cancer treatment. However, only recently has targeting the tumor microenvironment been explored as an option to selectively concentrate chemotherapeutic agents to the tumor site. In this regard, increasing evidence has demonstrated the potential importance the microenvironment plays in the tumorigenic events. Specifically, the extracellular matrix (ECM) represents a primary component of the tumor microenvironment. Recently, various studies [1-4] have shed light on poorly understood mechanisms that regulate how the ECM influences tumor cell proliferation, dissemination, invasion, and metastasis formation. Thus, strategies that implement specific targeting of the tumor microenvironment could usher in a new generation of therapeutic agents with a favorable impact on patient outcome.

Within this context, the ECM-associated enzyme lysyl oxidase (LOX) represents a promising candidate for selective drug delivery to the tumor site. This ECM-remodeling protein is overexpressed in primary and metastatic lesions of various tumors including breast, pancreas, and bone [5-12]. The spectrum of LOX activity is wide and includes the cross-linking of elastin and collagen fibers [13,14], modulation of the structure and stiffness of tumor ECM [15,16], and the regulation of cell migration and adhesion [17,18]. These findings have generated considerable interest in LOX involvement in tumor pathophysiology and its potential for cancer therapy. Previous studies demonstrated that metastatic growth from breast, prostate, and lung tumors could be slowed or arrested by the inhibition of LOX activity using local or systemic injection of antibodies, pro-peptides, or small molecules [11, 19-23].

Although promising results have been obtained using LOX-directed molecules, accumulation of these molecules in healthy organs has limited their clinical translation due to issues with significant toxicity and adverse side effects [24,25]. Recently, preliminary evidences of functionalizing the surface of poly(lactic-co-glycolic acid) PLGA-nanoparticles with a LOX inhibiting antibody has demonstrated promising results in suppressing cancer cell growth [26].

Therefore, there is still the need for improved carriers for chemotherapeutic drugs.

### SUMMARY OF THE INVENTION

In the field of oncology research, a deeper understanding of tumor biology has shed light on the role of environmental conditions surrounding cancer cells. In this regard targeting the tumor microenvironment has recently emerged as a new way to access this disease. The development of alternative strategies in tumor treatment rely on the unmet clinical need of improving therapeutic agents currently available which suffer from limited efficacy and systemic toxicity due to non-specific targeting. In this context, the nanotechnology approach represents a promising way to overcome these drawbacks with the use of nanoterapeutics designed to selectively concentrate drugs to the tumor site. As a result, tumor targeting represents one of the primary directives of nanotechnologies.

In the present invention, a lipid-based nanocarrier, which is widely used in the clinical setting, functionalized with a LOX antibody and loaded with a therapeutic agent, such as epirubicin, which represents one of the standard clinical treatment for breast cancer patients was engineered. The concentration of the anti-LOX used was strongly reduced in order to prevent *in vivo* toxicity and to obtain a translational result compared to previous work. *In vivo* analysis were designed to be near-patient treatment regimen. In the present invention a nano-drug which combines the intrinsic therapeutic activity of the LOX antibody together with its ability of selectively concentrate the therapeutic agent at the tumor site was developed. This delivery approach produces significant advantages in terms of increased therapeutic efficacy and reduced systemic toxicity.

In more details, in the present invention, a novel extracellular matrix (ECM)-targeting nanotherapeutic agent was engineered using a lipid-based nanoparticle chemically linked to an inhibitor of the ECM-related enzyme, lysyl oxidase 1 (LOX) that inhibits the crosslinking of elastin and collagen fibers. When loaded with a chemotherapeutic agent, such as epirubicin, superior inhibition of triple negative breast cancer (TNBC) cell growth both *in vitro* and *in vivo* was demonstrated. Moreover, *in vivo* results displayed minimal cytotoxicity and enhanced biocompatibility compared to free epirubicin. This all-in-one ECM-targeted chemotherapeutic nano-based therapy provides a key-enabling technology for the treatment of cancer, in particular TNBC.

Therefore the present invention provides a liposome comprising:
- a poly(ethylene glycol)-lipid (PEG-lipid) or a derivative thereof;
- cholesterol
- an anti-LOX antibody and
- a therapeutic agent.

Preferably the PEG-lipid or the PEG-lipid derivative is selected from the group consisting of: 1-oleoyl-2-hydroxy-sn-glycero-3-phosphocholine; 1-hexanoyl-2-hydroxy-sn-glycero-3-phosphocholine; 1-heptanoyl-2-hydroxy-sn-glycero-3-phosphocholine; 1-octanoyl-2-hydroxy-sn-glycero-3-phosphocholine; 1-nonanoyl-2-hydroxy-sn-glycero-3-phosphocholine; 1-decanoyl-2-hydroxy-sn-glycero-3-phosphocholine; 1-undecanoyl-2-hydroxy-sn-glycero-3-phosphocholine; 1-lauroyl-2-hydroxy-sn-glycero-3-phosphocholine; 1-tridecanoyl-2-hydroxy-sn-glycero-3-phosphocholine; 1-myristoyl-2-hydroxy-sn-glycero-3-phosphocholine; 1-pentadecanoyl-2-hydroxy-sn-glycero-3-phosphocholine; 1-palmitoyl-2-hydroxy-sn-glycero-3-phosphocholine; 1-heptadecanoyl-2-hydroxy-sn-glycero-3-phosphocholine; 1-(10Z-heptadecenoyl)-2-hydroxy-sn-glycero-3-phosphocholine; 1-hydroxy-2-oleoyl-sn-glycero-3-phosphocholine; 2-stearoyl-sn-glycero-3-phosphocholine; 2-stearoyl-sn-glycero-3-phosphocholine; 1-stearoyl-2-hydroxy-sn-glycero-3-phosphocholine; 1-nonadecanoyl-2-hydroxy-sn-glycero-3-phosphocholine; 1-arachidoyl-2-hydroxy-sn-glycero-3-phosphocholine; 1-behenoyl-2-hydroxy-sn-glycero-3-phosphocholine; 1-lignoceroyl-2-hydroxy-sn-glycero-3-phosphocholine; 1-hexacosanoyl-2-hydroxy-sn-glycero-3-phosphocholine; 1-myristoyl-2-palmitoyl-sn-glycero-3-phosphocholine; 1-myristoyl-2-stearoyl-sn-glycero-3-phosphocholine; 1-palmitoyl-2-myristoyl-sn-glycero-3-phosphocholine; 1-palmitoyl-2-stearoyl-sn-glycero-3-phosphocholine; 1-palmitoyl-2-oleoyl-glycero-3-phosphocholine; 1-palmitoyl-2-linoleoyl-sn-glycero-3-phosphocholine; 1-stearoyl-2-arachidonoyl-sn-glycero-3-phosphocholine; 1-stearoyl-2-docosahexaenoyl-sn-glycero-3-phosphocholine; 1-oleoyl-2-myristoyl-sn-glycero-3-phosphocholine; 1-oleoyl-2-myristoyl-sn-glycero-3-phosphocholine; 1-oleoyl-2-palmitoyl-sn-glycero-3-phosphocholine; 1-oleoyl-2-stearoyl-sn-glycero-3-phosphocholine; 1-oleoyl-2-stearoyl-sn-glycero-3-phosphocholine; 1-palmitoyl-2-acetyl-sn-glycero-3-phosphocholine; 1,2-dimyristoleoyl-sn-glycero-3-phosphocholine; 1,2-dimyristelaidoyl-sn-glycero-3-phosphocholine; 1,2-dipalmitoleoyl-sn-glycero-3-phosphocholine; 1,2-dipalmitelaidoyl-sn-glycero-3-phosphocholine; 1,2-dipalmitelaidoyl-sn-glycero-3-phosphocholine; 1,2-dipetroselenoyl-sn-glycero-3-phosphocholine; 1,2-dioleoyl-sn-glycero-3-phosphocholine; 1,2-dielaidoyl-sn-glycero-3-phosphocholine; 1,2-dielaidoyl-sn-glycero-3-phosphocholine; 1,2-dielaidoyl-sn-glycero-3-phosphocholine; 1,2-dilinoleoyl-sn-glycero-3-phosphocholine; 1,2-dilinolenoyl-sn-glycero-3-phosphocholine; 1,2-dieicosenoyl-sn-glycero-3-phosphocholine; 1,2-diarachidonoyl-sn-glycero-3-phosphocholine; 1,2-dierucoyl-sn-glycero-3-phosphocholine; 1,2-dinervonoyl-sn-glycero-3-phosphocholine; 1,2-didocosahexaenoyl-sn-glycero-3-phosphocholine; 1,2-dipropionyl-sn-glycero-3-phosphocholine; 1,2-dibutyryl-sn-glycero-3-phosphocholine; 1,2-dipentanoyl-sn-glycero-3-phosphocholine; 1,2-dihexanoyl-sn-glycero-3-phosphocholine; 1,2-diheptanoyl-sn-glycero-3-phosphocholine; 1,2-dioctanoyl-sn-glycero-3-phosphocholine; 1,2-dinonanoyl-sn-glycero-3-phosphocholine; 1,2-didecanoyl-sn-glycero-3-phosphocholine; 1,2-diundecanoyl-sn-glycero-3-phosphocholine; 1,2-dilauroyl-sn-glycero-3-phosphocholine; 1,2-ditridecanoyl-sn-glycero-3-phosphocholine; 1,2-dimyristoyl-sn-glycero-3-phosphocholine; 1,2-dipentadecanoyl-sn-glycero-3-phosphocholine; 1,2-dipalmitoyl-sn-glycero-3-phosphocholine; 1,2-diheptadecanoyl-sn-glycero-3-phosphocholine; 1,2-distearoyl-sn-glycero-3-phosphocholine; 1,2-dinonadecanoyl-sn-glycero-3-phosphocholine; 1,2-diarachidoyl-sn-glycero-3-phosphocholine; 1,2-diheneicosanoyl-sn-glycero-3-phosphocholine; 1,2-dibehenoyl-sn-glycero-3-phosphocholine; 1,2-ditricosanoyl-sn-glycero-3-phosphocholine; 1,2-dilignoceroyl-sn-glycero-3-phosphocholine; L-α-phosphatidylcholine (95%) (Egg, Chicken); L-α-Phosphatidylcholine (Egg, Chicken-60%) (Total Egg Phosphatide Extract); L-α-Phosphatidylcholine (Soy-40%); L-α-phosphatidylcholine (95%) (Soy); L-α-Phosphatidylcholine, 20% (Soy)(Soy Total Lipid Extract); L-α-lysophosphatidylcholine (Egg, Chicken); L-α-lysophosphatidylcholine (Egg, Chicken); L-α-phosphatidylcholine (Egg, Chicken); L-α-phosphatidylcholine (Egg, Chicken); L-α-phosphatidylcholine (Heart, Bovine); L-α-phosphatidylcholine (Brain, Porcine); L-α-phosphatidylcholine (Soy); L-α-phosphatidylcholine (Liver, Bovine); L-α-phosphatidylcholine, hydrogenated (Soy); L-α-phosphatidylcholine, hydrogenated (Egg, Chicken); L-α-lysophosphatidylcholine (Soy); 1,2-dipalmitoyl-sn-glycero-3-phosphoethanolamine-N-[methoxy(polyethylene glycol)-5000] (ammonium salt); 1,2-dimyristoyl-sn-glycero-3-phosphoethanolamine-N-[methoxy(polyethylene glycol)-5000] (ammonium salt); 1,2-distearoyl-sn-glycero-3-phosphoethanolamine-N-[methoxy(polyethylene glycol)-5000] (ammonium salt); 1,2-dioleoyl-sn-glycero-3-phosphoethanolamine-N-[methoxy(polyethylene glycol)-5000] (ammonium salt); 1,2-dipalmitoyl-sn-glycero-3-phosphoethanolamine-N-[methoxy(polyethylene glycol)-3000] (ammonium salt); 1,2-dimyristoyl-sn-glycero-3-phosphoethanolamine-N-[methoxy(polyethylene glycol)-3000] (ammonium salt); 1,2-distearoyl-sn-glycero-3-phosphoethanolamine-N-[methoxy(polyethylene glycol)-3000] (ammonium salt); 1,2-dioleoyl-sn-glycero-3-phosphoethanolamine-N-[methoxy(polyethylene glycol)-3000] (ammonium salt); 1,2-distearoyl-sn-glycero-3-phosphoethanolamine-N-[methoxy(polyethylene glycol)-2000] (ammonium salt); 1,2-dioleoyl-sn-glycero-3-phosphoethanolamine-N-[methoxy(polyethylene glycol)-2000] (ammonium salt); 1,2-dimyristoyl-sn-glycero-3-phosphoethanolamine-N-[methoxy(polyethylene glycol)-2000] (ammonium salt); 1,2-dipalmitoyl-sn-glycero-3-phosphoethanolamine-N-[methoxy(polyethylene glycol)-2000] (ammonium salt); 1,2-dipalmitoyl-sn-glycero-3-phosphoethanolamine-N-[methoxy(polyethylene glycol)-1000] (ammonium salt); 1,2-dimyristoyl-sn-glycero-3-phosphoethanolamine-N-[methoxy(polyethylene glycol)-1000] (ammonium salt); 1,2-distearoyl-sn-glycero-3-phosphoethanolamine-N-[methoxy(polyethylene glycol)-1000] (ammonium salt); 1,2-dimyristoyl-sn-glycero-3-phosphoethanolamine-N-[methoxy(polyethylene glycol)-1000] (ammonium salt); 1,2-distearoyl-sn-glycero-3-phosphoethanolamine-N-[methoxy(polyethylene glycol)-1000] (ammonium salt); 1,2-dioleoyl-sn-glycero-3-phosphoethanolamine-N-[methoxy(polyethylene glycol)-1000] (ammonium salt); 1,2-dipalmitoyl-sn-glycero-3-phosphoethanolamine-N-[methoxy(polyethylene glycol)-750] (ammonium salt); 1,2-dimyristoyl-sn-glycero-3-phosphoethanolamine-N-[methoxy(polyethylene glycol)-750] (ammonium salt); 1,2-distearoyl-sn-glycero-3-phosphoethanolamine-N-[methoxy(polyethylene glycol)-750] (ammonium salt); 1,2-dioleoyl-sn-glycero-3-phosphoethanolamine-N-[methoxy(polyethylene glycol)-750] (ammonium salt); 1,2-dipalmitoyl-sn-glycero-3-phosphoethanolamine-N-[methoxy(polyethylene glycol)-550] (ammonium salt); 1,2-dimyristoyl-sn-glycero-3-phosphoethanolamine-N-[methoxy(polyethylene glycol)-550] (ammonium salt); 1,2-dimyristoyl-sn-glycero-3-phosphoethanolamine-N-[methoxy(polyethylene glycol)-550] (ammonium salt); 1,2-distearoyl-sn-glycero-3-phosphoethanolamine-N-[methoxy(polyethylene glycol)-550] (ammonium salt); 1,2-dioleoyl-sn-glycero-3-phosphoethanolamine-N-[methoxy(polyethylene glycol)-550] (ammonium salt); 1,2-dipalmitoyl-sn-glycero-3-phosphoethanolamine-N-[methoxy(polyethylene glycol)-350] (ammonium salt); 1,2-dimyristoyl-sn-glycero-3-phosphoethanolamine-N-[methoxy(polyethylene glycol)-350] (ammonium salt); 1,2-dimyristoyl-sn-glycero-3-phosphoethanolamine-N-[methoxy(polyethylene glycol)-350] (ammonium salt); 1,2-distearoyl-sn-glycero-3-phosphoethanolamine-N-[methoxy(polyethylene glycol)-350] (ammonium salt); 1,2-dioleoyl-sn-glycero-3-phosphoethanolamine-N-[methoxy(polyethylene glycol)-350] (ammonium salt); 1,2-distearoyl-sn-glycero-3-phosphoethanolamine-N-[succinyl(polyethylene glycol)-2000] (ammonium salt); 1,2-distearoyl-sn-glycero-3-phosphoethanolamine-N-[cyanur(polyethylene glycol)-2000] (ammonium salt); 1,2-distearoyl-sn-glycero-3-phosphoethanolamine-N-[folate(polyethylene glycol)-5000] (ammonium salt); 1,2-distearoyl-sn-glycero-3-phosphoethanolamine-N-[folate(polyethylene glycol)-2000] (ammonium salt); 1,2-distearoyl-sn-glycero-3-phosphoethanolamine-N-[maleimide(polyethylene glycol)-2000] (ammonium salt); 1,2-distearoyl-sn-glycero-3-phosphoethanolamine-N-[PDP(polyethylene glycol)-2000] (ammonium salt); 1,2-distearoyl-sn-glycero-3-phosphoethanolamine-N-[amino(polyethylene glycol)-2000] (ammonium salt); 1,2-distearoyl-sn-glycero-3-phosphoethanolamine-N-[amino(polyethylene glycol)-2000] (ammonium salt); 1,2-distearoyl-sn-glycero-3-phosphoethanolamine-N-[biotinyl(polyethylene glycol)-2000] (ammonium salt); 1,2-distearoyl-sn-glycero-3-phosphoethanolamine-N-[10-(trimethoxysilyl)undecanamide(polyethylene glycol)-2000] (triethylammonium salt); 1,2-distearoyl-sn-glycero-3-phosphoethanolamine-N-[amino(polyethylene glycol)-5000] (ammonium salt); Bis(1,2-distearoyl-sn-glycero-3-phosphoethanolamine)-N-[(polyethylene glycol)-2000] (sodium salt); 1,2-distearoyl-sn-glycero-3-phosphoethanolamine-N-[carboxy(polyethylene glycol)-2000] (sodium salt); 1,2-distearoyl-sn-glycero-3-phosphoethanolamine-N-[square(polyethylene glycol)-2000] (sodium salt); 1,2-distearoyl-sn-glycero-3-phosphoethanolamine-N-[maleimide(polyethylene glycol)-5000] (ammonium salt); 1,2-distearoyl-sn-glycero-3-phosphoethanolamine-n-[dibenzocyclooctyl(polyethylene glycol)-5000] (ammonium salt); 1,2-distearoyl-sn-glycero-3-phosphoethanolamine-N-[azido(polyethylene glycol)-5000] (ammonium salt); 1,2-distearoyl-sn-glycero-3-phosphoethanolamine-N-[azido(polyethylene glycol)-2000] (ammonium salt); 1,2-distearoyl-sn-glycero-3-phosphoethanolamine-N-[dibenzocyclooctyl(polyethylene glycol)-2000] (ammonium salt); 1,2-dipalmitoyl-sn-glycero-3-phosphoethanolamine-N-[azido(polyethylene glycol)-2000 (ammonium salt); 1,2-dioleoyl-sn-glycero-3-phosphoethanolamine-N-[azido(polyethylene glycol)-2000] (ammonium salt); 1,2-dioleoyl-sn-glycero-3-phosphoethanolamine-N-[azido(polyethylene glycol)-2000] (ammonium salt); 1,2-dioleoyl-sn-glycero-3-phosphoethanolamine-N-[amino(polyethylene glycol)-2000] (ammonium salt); 1,2-dioleoyl-sn-glycero-3-phosphoethanolamine-N-[carboxy(polyethylene glycol)-2000] (sodium salt); Cholesterol-(polyethylene glycol-600); 1,2-dimyristoyl-rac-glycero-3-methoxypolyethylene glycol-2000; distearoyl-rac-glycerol-PEG2K; 1,2-bis(10,12-tricosadiynoyl)-sn-glycero-3-phosphoethanolamine; 1-palmitoyl-2-(10,12-tricosadiynoyl)-sn-glycero-3-phosphocholine; 1-palmitoyl-2-(10,12-tricosadiynoyl)-sn-glycero-3-phosphoethanolamine; 1,2-bis(10,12-tricosadiynoyl)-sn-glycero-3-phosphocholine; N-octanoyl-sphingosine-1-{succinyl[methoxy(polyethylene glycol)2000]}; N-palmitoyl-sphingosine-1-{succinyl[methoxy(polyethylene glycol)2000]}; N-octanoyl-sphingosine-1-{succinyl[methoxy(polyethylene glycol)5000]}; N-palmitoyl-sphingosine-1-{succinyl[methoxy(polyethylene glycol)5000]}; N-octanoyl-sphingosine-1-{succinyl[methoxy(polyethylene glycol)750]}; N-palmitoyl-sphingosine-1-{succinyl[methoxy(polyethylene glycol)750]}

Preferably the PEG derivative is selected from the group consisting of: FUMONISIN B2, Tetraethyleneglycol monododecyl ether, Polyethylene glycol monolaurate, Poly(ethylene glycol) dimethacrylate, Methoxypolyethylene glycols, Poly(ethylene glycol), heptaethylene glycol monododecyl ether, Poly(ethylene glycol) diacrylate, Polyethylene glycol dimethyl ether, hexaethylene glycol monomethyl ether, alkyl ketene dimer, polyethylene glycol monolaurate n(=:)10, poly(ethylene glycol) methyl ether methacrylate, diethylene glycol monolaurate, octaethylene glycol monomethyl ether, Tetraethyleneglycol monooctyl ether, poly(ethylene glycol succinate), h2n-peg-nh2, polyethyleneglycol 600 monooctyl ether, mono-methyl polyethylene glycol 5'000 2-maleimidoethyl ether, poly(ethylene glycol) divinyl ether, o,o'-bis(3-aminopropyl)polyethylene glycol 1'500, aminopolyethylene glycol 5'000 monomethyl ether, c14e6, polyethylene glycol stearylamine, polyethylene glycol 5'000 monomethyl, allyloxypolyethyleneglycol, poly(ethylene glycol) methyl ether acrylate, poly(ethylene glycol) bis(carboxymethyl) ether, hexaethylene glycol monohexadecyl ether, C10E3, C18E3, Polyethylene Glycol 600 Dioleate, Diethylene glycol diglycidyl ether, Thickening emulsifier agent M, polyethylene glycol-bound ruthenium carbene complex, mono-methyl polyethylene glycol 5'000 succinate n-succinimidyl ester, mono-methyl polyethylene glycol 5'000acetic acid n-succinimidyl ester, poly(ethylene glycol) 4-nonylphenyl 3-sulfopropyl ether, potassium salt, polyethylene glycol 4000 dimesylate, diethylene glycol mono-methacrylate, tetraethylene glycol monotetradecyl ether, Macergo 8000, heptaethylene glycol, Peginterferon alfa-2b, C10E9, polyethylene glycol trimethylnonyl ether, C10E5, PEG 400-6000, Tetraethyleneglycol monodecyl ether, poly(ethylene glycol) butyl ether, methoxypoly(ethylene glycol) aldehyde, hexaethylene glycol monodecyl ether, Polyethylene glycol 800, Polyethylene glycol distearate, dimethyl polyethylene glycol, Polyoxyethylene dilaurate, 4-ArM poly(ethylene glycol) Methacrylate, Methoxypoly(ethylene glycol) isocyanate, Y-shape poly(ethylene glycol) acrylate, Y-shape poly(ethylene glycol) carboxylic acid, α-Acryloyl-ω-azido poly(ethylene glycol), α-Acryloyl-ω-succiniMidyl poly(ethylene glycol), α-Carboxyl-ω-aMino poly(ethylene glycol), α-Carboxyl-ω-Methacryloyl poly(ethylene glycol), Polyethylene glycol di-p-toluenesulfonate, M.W. 3,400, Poly(ethylene glycol) graft glycidyl ether, α-SucciniMidyl-ω-hydroxyl poly(ethylene glycol), Macergo 4000, Nonylphenoxypolyethanol-iodine complex, 2-arM Methoxypoly(ethylene glycol) succiniMidyl ester (Lysine), 4-arM Poly(ethylene glycol) aMine, Novel 2-arM Methoxypoly(ethylene glycol) alkyne, Y-shape poly(ethylene glycol) Methacrylate, α,ω-DiforMyl poly(ethylene glycol), α-Allyl-ω-forMyl poly(ethylene glycol), α-MaleiMidyl-ω-hydroxyl Poly(ethylene glycol), Polyethylene glycol di-p-toluenesulfonate, M.W. 1,000, triethylene glycol monotetradecyl ether, methoxypolyethyleneglycolglycidylether, Poly(oxy-1,2-ethanediyl), .alpha.-(2-methyl-1-oxo-2-propenyl)-.omega.-(phosphonooxy), peginterferon alpha-2a, 4-arM Poly(ethylene glycol) succiniMidyl este, Novel 2-arM Methoxypoly(ethylene glycol), Y-shape poly(ethylene glycol) aldehyde, Y-shape poly(ethylene glycol) glycidyl ether, α-Allyl-ω-aMino poly(ethylene glycol), α-Carboxyl-ω-azido poly(ethylene glycol), α-ForMyl-ω-aMino poly(ethylene glycol), α-Azido-ω-aMino poly(ethylene glycol), poly(ethylene glycol) bis(2-ethylhexanoate), 4-arM Poly(ethylene glycol) alkyne, Novel 2-arM Methoxypoly(ethylene glycol) thio, α,ω-Diazido poly(ethylene glycol), α-Acryloyl-ω-allyl poly(ethylene glycol), α-Acryloyl-ω-Maleinidyl poly(ethylene glycol), α-Carboxyl-ω-hydroxyl poly(ethylene glycol), α-Methacryloyl-ω-glycidyl poly(ethylene glycol), Poly(ethylene glycol) graft alkyne, α-Methacryloyl-ω-hydroxy poly(ethylene glycol) Preferably the PEG-lipid is selected from the group consisting of: 1,2-distearoyl-sn-glycero-3-phosphocholine (DSPC), diacyl-phosphatidylethanolamine-N-[methoxy(polyethene glycol)]; distearoyl- phosphatidylethanolamine-N-[methoxy(polyethylene glycol)-2000] (DSPE-PEG-2000); and distearoyl-phosphatidylethanolamine-N-[methoxy(polyethylene glycol)-5000] (DSPE-PEG- 5000). Preferably the PEG-lipid or the PEG-lipid derivative may be a mixture of different PEGs.

Generally, PEG refers to a polyethylene glycol, a linear, water-soluble polymer of ethylene PEG repeating units with two terminal hydroxyl groups. PEGs are classified by their molecular weights; for example, PEG 2000 has an average molecular weight of about 2,000 Daltons, and PEG 5000 has an average molecular weight of about 5,000 Daltons. PEGs are commercially available from Sigma Chemical Co., Genzyme Pharmaceuticals, and other companies and include, for example, the following: methylpolyethyleneglycol-1,2-distearoyl-phosphatidyl ethanolamine conjugate (MPEG-2000-DSPE); monomethoxypolyethylene glycol (MPEG-OH), monomethoxypolyethylene glycol-succinate (MPEG-S), monomethoxypolyethylene glycol-succinimidyl succinate (MPEG-S-NHS), monomethoxypolyethylene glycol-amine (MPEG-NH2), monomethoxypolyethylene glycol-tresylate (MPEG-TRES), and monomethoxypolyethylene glycol-imidazolyl-carbonyl (MPEG-IM), or mixtures thereof.

In various embodiments, the PEG is a polyethylene glycol with an average molecular weight of about 550 to about 10,000 Daltons and is optionally substituted by alkyl, alkoxy, acyl or aryl. In an embodiment, the PEG is substituted with methyl at the terminal hydroxyl position. In another embodiment, the PEG has an average molecular weight of about 750 to about 5,000 Daltons, more preferably, of about 1,000 to about 5,000 Daltons, more preferably about 1,500 to about 3,000 Daltons and, even more preferably, of about 2,000 Daltons or of about 750 Daltons. The PEG can be optionally substituted with alkyl, alkoxy, acyl or aryl. In a preferred embodiment, the terminal hydroxyl group is substituted with a methoxy or methyl group. The PEGylated liposomes of the invention may also comprise cholesterol, cholesterol derivatives, or combinations of the derivatives or cholesterol. Generally, the cholesterol component of a PEGylated liposome provides additional stability to the liposome structure.

The PEGylated liposomes of the invention may also comprise phospholipids. Phospholipids that may be used to create PEGylated liposomes are, without limitation, 1,2-dimyristroyl-sn-glycero-3-phosphocholine, 1,2-dilauroyl-sn-glycero-3-phosphocholine, 1,2-distearoyl-sn-glycero-3-phosphocholine, 1,2-dimyristoyl-sn-glycero-3-phosphoethanolamine, 1,2-dipalmitoyl-sn-glycero-3-phosphoethanolamine, 1,2-dioleoyl-sn-glycero-3-phosphate monosodium salt, 1,2-dipalmitoyl-sn-glycero-3[phosphor-rac-(1-glycerol)]sodium salt, 1,2-dimyristoyl-sn-glycero-3-[phospho-L-serine]sodium salt, 1,2-dioleoyl-sn-glycero-3-phosphoethanolamine-N-glutaryl sodium salt and 1,1',2,2'-tetramyristoyl cardiolipin ammonium salt, or mixtures thereof.

Preferably the anti-LOX antibody is functionalized through conjugation to the carbonyl function group via the PEG termini.

In the present invention the anti-LOX antibody may be any known anti-LOX antibody such as ab 31238 or any other LOX inhibitory antibody.

In a preferred embodiment, the therapeutic agent is a chemotherapy, preferably said chemotherapy is selected from the group consisting of: a chemotherapeutic agent, an immunotherapeutic agent, a targeted therapy and a radiometabolic agent alone or in combination.

Preferably the therapeutic agent is selected from the group consisting of:
Abemaciclib, Abiraterone Acetate, Abraxane (Paclitaxel Albumin-stabilized Nanoparticle Formulation), Acalabrutinib, Actemra (Tocilizumab), Adcetris (Brentuximab Vedotin), Ado-Trastuzumab Emtansine, Adriamycin (Doxorubicin Hydrochloride), Afatinib Dimaleate, Afinitor (Everolimus), Aldara (Imiquimod), Aldesleukin, Alecensa (Alectinib), Alectinib, Alemtuzumab, Alimta (Pemetrexed Disodium), Aliqopa (Copanlisib Hydrochloride), Alkeran for Injection (Melphalan Hydrochloride), Alkeran Tablets (Melphalan), Alpelisib, Alunbrig (Brigatinib), Ameluz (Aminolevulinic Acid Hydrochloride), Amifostine, Aminolevulinic Acid Hydrochloride, Anastrozole, Apalutamide, Aredia (Pamidronate Disodium), Arimidex (Anastrozole), Aromasin (Exemestane), Arranon (Nelarabine), Arsenic Trioxide, Arzerra (Ofatumumab), Asparaginase Erwinia chrysanthemi, Asparlas (Calaspargase Pegol-mknl), Atezolizumab, Avastin (Bevacizumab), Avelumab, Axicabtagene Ciloleucel, Axitinib, Azacitidine, Azedra (lobenguane I 131), Balversa (Erdafitinib), Bavencio (Avelumab), Beleodaq (Belinostat), Belinostat, Bendamustine Hydrochloride, Bendeka (Bendamustine Hydrochloride), Besponsa (Inotuzumab Ozogamicin), Bevacizumab, Bexarotene, Bicalutamide, BiCNU (Carmustine), Binimetinib, Bleomycin Sulfate, Blinatumomab, Blincyto (Blinatumomab), Bortezomib, Bosulif (Bosutinib), Bosutinib, Braftovi (Encorafenib), Brentuximab Vedotin, Brigatinib, Busulfan, Busulfex (Busulfan), Cabazitaxel, Cablivi (Caplacizumab-yhdp), Cabometyx (Cabozantinib-S-Malate), Cabozantinib-S-Malate, Calaspargase Pegol-mknl, Calquence (Acalabrutinib), Campath (Alemtuzumab), Camptosar (Irinotecan Hydrochloride), Capecitabine, Caplacizumab-yhdp,Carac (Fluorouracil--Topical), Carboplatin, Carfilzomib, Carmustine, Carmustine Implant, Casodex (Bicalutamide), Cemiplimab-rwlc, Ceritinib, Cerubidine (Daunorubicin Hydrochloride), Cervarix (Recombinant HPV Bivalent Vaccine), Cetuximab, Chlorambucil, Cisplatin, Cladribine, Clofarabine, Clolar (Clofarabine), Cobimetinib, Cometriq (Cabozantinib-S-Malate), Copanlisib Hydrochloride, Cosmegen (Dactinomycin), Cotellic (Cobimetinib), Crizotinib, Cyclophosphamide, Cyramza (Ramucirumab), Cytarabine, Cytarabine Liposome, Dabrafenib Mesylate, Dacarbazine, Dacogen (Decitabine), Dacomitinib, Dactinomycin, Daratumumab, Darzalex (Daratumumab), Dasatinib, Daunorubicin Hydrochloride, Daunorubicin Hydrochloride and Cytarabine Liposome, Daurismo (Glasdegib Maleate), Decitabine, Defibrotide Sodium, Defitelio (Defibrotide Sodium), Degarelix, Denileukin Diftitox, Denosumab, DepoCyt (Cytarabine Liposome), Dexamethasone, Dexrazoxane Hydrochloride, Dinutuximab, Docetaxel, Doxil (Doxorubicin Hydrochloride Liposome), Doxorubicin Hydrochloride, Doxorubicin Hydrochloride Liposome, Durvalumab, Duvelisib, Efudex (Fluorouracil--Topical), Eligard (Leuprolide Acetate), Elitek (Rasburicase), Ellence (Epirubicin Hydrochloride), Elotuzumab, Eloxatin (Oxaliplatin), Eltrombopag Olamine, Elzonris (Tagraxofusp-erzs), Emapalumab-lzsg, Empliciti (Elotuzumab), Enasidenib Mesylate, Encorafenib, Enzalutamide, Epirubicin Hydrochloride, Erbitux (Cetuximab), Erdafitinib, Eribulin Mesylate, Erivedge (Vismodegib), Erleada (Apalutamide), Erlotinib Hydrochloride, Erwinaze (Asparaginase Erwinia chrysanthemi), Ethyol (Amifostine), Etopophos (Etoposide Phosphate), Etoposide, Etoposide Phosphate, Everolimus, Evista (Raloxifene Hydrochloride), Evomela (Melphalan Hydrochloride), Exemestane, 5-FU (Fluorouracil Injection), 5-FU (Fluorouracil--Topical), Fareston (Toremifene), Farydak (Panobinostat), Faslodex (Fulvestrant), Femara (Letrozole), Firmagon (Degarelix), Fludarabine Phosphate, Fluoroplex (Fluorouracil--Topical), Fluorouracil Injection, Fluorouracil-Topical, Flutamide, Folotyn (Pralatrexate), Fostamatinib Disodium, Fulvestrant, Fusilev (Leucovorin Calcium), Gamifant (Emapalumab-lzsg), Gardasil (Recombinant HPV Quadrivalent Vaccine), Gardasil 9 (Recombinant HPV Nonavalent Vaccine), Gazyva (Obinutuzumab), Gefitinib, Gemcitabine Hydrochloride, Gemtuzumab Ozogamicin, Gemzar (Gemcitabine Hydrochloride), Gilotrif (Afatinib Dimaleate), Gilteritinib Fumarate, Glasdegib Maleate, Gleevec (Imatinib Mesylate), Gliadel Wafer (Carmustine Implant), Glucarpidase, Goserelin Acetate, Halaven (Eribulin Mesylate), Hemangeol (Propranolol Hydrochloride), Herceptin Hylecta (Trastuzumab and Hyaluronidase-oysk), Herceptin (Trastuzumab), HPV Bivalent Vaccine, HPV Nonavalent Vaccine, Recombinant, HPV Quadrivalent Vaccine, Hycamtin (Topotecan Hydrochloride), Hydrea (Hydroxyurea), Hydroxyurea, Ibrance (Palbociclib), Ibritumomab Tiuxetan, Ibrutinib, Iclusig (Ponatinib Hydrochloride), Idamycin PFS (Idarubicin Hydrochloride), Idarubicin Hydrochloride, Idelalisib, Idhifa (Enasidenib Mesylate), Ifex (Ifosfamide), Ifosfamide, IL-2 (Aldesleukin), Imatinib Mesylate, Imbruvica (Ibrutinib), Imfinzi (Durvalumab), Imiquimod, Imlygic (Talimogene Laherparepvec), Inlyta (Axitinib), Inotuzumab Ozogamicin, Interferon Alfa-2b, Interleukin-2 (Aldesleukin), Intron A (Recombinant Interferon Alfa-2b), lobenguane I 131, Ipilimumab, Iressa (Gefitinib), Irinotecan Hydrochloride, Irinotecan Hydrochloride Liposome, Istodax (Romidepsin), Ivosidenib, Ixabepilone, Ixazomib Citrate, Ixempra (Ixabepilone), Jakafi (Ruxolitinib Phosphate), Jevtana (Cabazitaxel), Kadcyla (Ado-Trastuzumab Emtansine), Kepivance (Palifermin), Keytruda (Pembrolizumab), Kisqali (Ribociclib), Kymriah (Tisagenlecleucel), Kyprolis (Carfilzomib), Lanreotide Acetate, Lapatinib Ditosylate, Larotrectinib Sulfate, Lartruvo (Olaratumab), Lenalidomide, Lenvatinib Mesylate, Lenvima (Lenvatinib Mesylate), Letrozole, Leucovorin Calcium, Leukeran (Chlorambucil), Leuprolide Acetate, Levulan Kerastik (Aminolevulinic Acid Hydrochloride), Libtayo (Cemiplimab-rwlc), Lomustine, Lonsurf (Trifluridine and Tipiracil Hydrochloride), Lorbrena (Lorlatinib), Lorlatinib, Lumoxiti (Moxetumomab Pasudotox-tdfk), Lupron (Leuprolide Acetate), Lupron Depot (Leuprolide Acetate), Lutathera (Lutetium Lu 177-Dotatate), Lutetium (Lu 177-Dotatate), Lynparza (Olaparib), Marqibo (Vincristine Sulfate Liposome), Matulane (Procarbazine Hydrochloride), Mechlorethamine Hydrochloride, Megestrol Acetate, Mekinist (Trametinib), Mektovi (Binimetinib), Melphalan, Melphalan Hydrochloride, Mercaptopurine, Mesna, Mesnex (Mesna), Methotrexate, Methylnaltrexone Bromide, Midostaurin, Mitomycin C, Mitoxantrone Hydrochloride, Mogamulizumab-kpkc, Moxetumomab Pasudotox-tdfk, Mustargen (Mechlorethamine Hydrochloride), Mvasi (Bevacizumab), Myleran (Busulfan), Mylotarg (Gemtuzumab Ozogamicin), Nanoparticle Paclitaxel (Paclitaxel Albumin-stabilized Nanoparticle Formulation), Navelbine (Vinorelbine Tartrate), Necitumumab, Nelarabine, Neratinib Maleate, Nerlynx (Neratinib Maleate), Nexavar (Sorafenib Tosylate), Nilandron (Nilutamide), Nilotinib, Nilutamide, Ninlaro (Ixazomib Citrate), Niraparib Tosylate Monohydrate, Nivolumab, Nplate (Romiplostim), Obinutuzumab, Octreotide, Octreotide Acetate, Odomzo (Sonidegib), Ofatumumab, Olaparib, Olaratumab, Omacetaxine Mepesuccinate, Oncaspar (Pegaspargase), Onivyde (Irinotecan Hydrochloride Liposome), Ontak (Denileukin Diftitox), Opdivo (Nivolumab), Osimertinib Mesylate, Oxaliplatin, Paclitaxel, Paclitaxel Albumin-stabilized Nanoparticle Formulation, Palbociclib, Palifermin, Pamidronate Disodium, Panitumumab, Panobinostat, Pazopanib Hydrochloride, Pegaspargase, Pegfilgrastim, Peginterferon Alfa-2b, PEG-Intron (Peginterferon Alfa-2b), Pembrolizumab, Pemetrexed Disodium, Perjeta (Pertuzumab), Pertuzumab, Piqray (Alpelisib), Plerixafor, Polatuzumab Vedotin-piiq, Polivy (Polatuzumab Vedotin-piiq), Pomalidomide, Pomalyst (Pomalidomide), Ponatinib Hydrochloride, Portrazza (Necitumumab), Poteligeo (Mogamulizumab-kpkc) Pralatrexate, Prednisone, Procarbazine Hydrochloride, Proleukin (Aldesleukin), Prolia (Denosumab), Propranolol Hydrochloride, Provenge (Sipuleucel-T), Purinethol (Mercaptopurine), Purixan (Mercaptopurine), Radium 223 Dichloride, Raloxifene Hydrochloride, Ramucirumab, Rasburicase, Ravulizumab-cwvz, Recombinant Human Papillomavirus (HPV) Bivalent Vaccine, Recombinant Human Papillomavirus (HPV) Nonavalent Vaccine, Recombinant Human Papillomavirus (HPV) Quadrivalent Vaccine, Recombinant Interferon Alfa-2b, Regorafenib, Relistor (Methylnaltrexone Bromide), Retacrit (Epoetin Alfa), Revlimid (Lenalidomide), Rheumatrex (Methotrexate), Ribociclib, Rituxan (Rituximab), Rituxan Hycela (Rituximab and Hyaluronidase Human), Rituximab, Rituximab and Hyaluronidase Human, Rolapitant Hydrochloride, Romidepsin, Romiplostim, Rubidomycin (Daunorubicin Hydrochloride), Rubraca (Rucaparib Camsylate), Rucaparib Camsylate, Ruxolitinib Phosphate, Rydapt (Midostaurin), Sclerosol Intrapleural Aerosol (Talc), Siltuximab, Sipuleucel-T, Somatuline Depot (Lanreotide Acetate), Sonidegib, Sorafenib Tosylate, Sprycel (Dasatinib), Sterile Talc Powder (Talc), Steritalc (Talc), Stivarga (Regorafenib), Sunitinib Malate, Sutent (Sunitinib Malate), Sylatron (Peginterferon Alfa-2b), Sylvant (Siltuximab), Synribo (Omacetaxine Mepesuccinate), Tabloid (Thioguanine), Tafinlar (Dabrafenib Mesylate), Tagraxofusp-erzs, Tagrisso (Osimertinib Mesylate), Talazoparib Tosylate, Talc, Talimogene Laherparepvec, Talzenna (Talazoparib Tosylate), Tamoxifen Citrate, Tarceva (Erlotinib Hydrochloride), Targretin (Bexarotene), Tasigna (Nilotinib), Tavalisse (Fostamatinib Disodium), Taxol (Paclitaxel), Taxotere (Docetaxel), Tecentriq (Atezolizumab), Temodar (Temozolomide), Temozolomide, Temsirolimus, Thalidomide, Thalomid (Thalidomide), Thioguanine, Thiotepa, Tibsovo (Ivosidenib), Tisagenlecleucel, Tocilizumab, Tolak (Fluorouracil-Topical), Topotecan Hydrochloride, Toremifene, Torisel (Temsirolimus), Totect (Dexrazoxane Hydrochloride), Trabectedin, Trametinib, Trastuzumab, Trastuzumab and Hyaluronidase-oysk, Treanda (Bendamustine Hydrochloride), Trexall (Methotrexate), Trifluridine and Tipiracil Hydrochloride, Trisenox (Arsenic Trioxide), Tykerb (Lapatinib Ditosylate), Ultomiris (Ravulizumab-cwvz), Unituxin (Dinutuximab), Uridine Triacetate, Valrubicin, Valstar (Valrubicin), Vandetanib, Vectibix (Panitumumab), Velcade (Bortezomib), Vemurafenib, Venclexta (Venetoclax), Venetoclax, Verzenio (Abemaciclib), Vidaza (Azacitidine), Vinblastine Sulfate, Vincristine Sulfate, Vincristine Sulfate Liposome, Vinorelbine Tartrate, Vismodegib, Vistogard (Uridine Triacetate), Vitrakvi (Larotrectinib Sulfate), Vizimpro (Dacomitinib), Voraxaze (Glucarpidase), Vorinostat, Votrient (Pazopanib Hydrochloride), Vyxeos (Daunorubicin Hydrochloride and Cytarabine Liposome), Xalkori (Crizotinib), Xeloda (Capecitabine), Xgeva (Denosumab), Xofigo (Radium 223 Dichloride), Xospata (Gilteritinib Fumarate), Xtandi (Enzalutamide), Yervoy (Ipilimumab), Yescarta (Axicabtagene Ciloleucel), Yondelis (Trabectedin), Zaltrap (Ziv-Aflibercept), Zejula (Niraparib Tosylate Monohydrate), Zelboraf (Vemurafenib), Zevalin (Ibritumomab Tiuxetan), Zinecard (Dexrazoxane Hydrochloride), Ziv-Aflibercept, Zoladex (Goserelin Acetate), Zoledronic Acid, Zolinza (Vorinostat), Zometa (Zoledronic Acid), Zydelig (Idelalisib), Zykadia (Ceritinib), Zytiga (Abiraterone Acetate), VAL-083 (dianhydrogalactitol), Vintafolide, 90Y-DOTATOC (yttrium (90Y) edotreotide), DOTA-lanreotide (DOTALAN), Somatostatin Analogues.

Preferably, said liposome has a Z average from 150 to 250 nm. Preferably, said liposome has or a Pdi from 0.02 to 0.2. Preferably, said liposome has a Z Potential from -20 to -30 mV. Preferably, said liposome has a EE% from 40 to 60%.

In a preferred embodiment the anti-LOX antibody:PEG-lipid ratio is from 1:100 to 1:5000, preferably from 1:300 to 1:1000, preferably 1:500.

It is an object of the invention a formulation comprising the liposome as defined above and a pharmaceutically-acceptable excipient.

Preferably the pharmaceutically-acceptable excipient is selected from the group consisting of: alpha-tocopherol, dl-, acacia, acesulfame, acesulfame potassium, acetic acid, acrylates copolymer, adipic acid, albumin aggregated, alcohol, alginic acid, aluminum polyester, ammonium acetate, ammonium sulfate, anethole, anhydrous citric acid, anhydrous dextrose, anhydrous lactose, anhydrous trisodium citrate, arginine, ascorbic acid, aspartic acid, barium sulfate, benzalkonium chloride, benzenesulfonic acid, benzethonium chloride, benzoic acid, benzyl alcohol, benzyl chloride, betadex sulfobutyl ether sodium, boric acid, butylated hydroxyanisole, butylated hydroxytoluene, butylparaben, caffeine, calcium, calcium carbonate, calcium chloride, calcium hydroxide, calcium saccharate, caldiamide sodium, caramel, carbomer 940, carbomer copolymer type b (allyl pentaerythritol crosslinked), carbon dioxide, carboxymethylcellulose, carboxymethylcellulose, carboxymethylcellulose sodium, carrageenan, castor oil, ceteth-20, chlorobutanol, chlorobutanol hemihydrate, cholesterol, citric acid monohydrate, coconut acid, copovidone k25-31, corn oil, cottonseed oil, creatinine, croscarmellose sodium, crospovidone, cyclomethicone, cysteine hydrochloride, detosu/triethylene glycol/triethylene glycol polyglycolide copolymer, dextran, dextran 40, dextrose, diatrizoic acid, dibasic potassium phosphate, diethanolamine, dimethyl sulfoxide, dipalmitoylphosphatidylglycerol, dl-, disodium citrate sesquihydrate, disodium hydrogen citrate, docusate sodium, edetate calcium disodium, edetate disodium, edetate disodium anhydrous, edetate trisodium, edetic acid, egg phospholipids, ethylcellulose, ethylene-propylene copolymer, ethylenediamine, ferric oxide red, intravenous, galactose, gelatin, gentisic acid, gluceptate sodium, gluconolactone, glycerin, glyceryl palmitostearate, glycine, glycine hydrochloride, high density polyethylene, histidine, hyaluronate sodium, hydrochloric acid, hydrogen peroxide, hydrolyzed soy protein (enzymatic; 2000 mw), hydroxyethyl cellulose, hydroxyethyl cellulose (2000 mpa.s at 1%), hydroxyethylpiperazine ethane sulfonic acid, hydroxypropyl betadex, hydroxypropyl cellulose (110000 wamw), hydroxypropyl cellulose (1600000 wamw), hypromellose 2910 (4000 mpa.s), hypromelloses, icodextrin, isoleucine, isopropyl alcohol, isotonic sodium chloride solution, kaolin, lactic acid, lactic acid, dl-, lactobionic acid, lactose, lactose monohydrate, laureth sulfate, lauric diethanolamide, lecithin, leucine, levulinic acid, light mineral oil, lysine, lysine acetate, magnesium chloride, magnesium stearate, maleic acid, maltose monohydrate, mannitol, medium-chain triglycerides, meglumine, menthol, metacresol, metaphosphoric acid, methanesulfonic acid, methyl laurate, methylcellulose, methylparaben, methylparaben sodium, microcrystalline cellulose, miripirium chloride, mono and diglyceride, monothioglycerol, multisterol extract, myristyl alcohol, n,n-dimethylacetamide, niacinamide, nitric acid, nonoxynol-40, octisalate, octyldodecanol, oleic acid, oleyl alcohol, oleyl oleate, palmitic acid, peanut oil, peg-60 hydrogenated castor oil, pentadecalactone, pentasodium pentetate, pentetic acid, petrolatum, phenol, phenylalanine, phenylethyl alcohol, phospholipid, phosphoric acid, phosphoric acid, poloxamer 188, poly(dl-lactic-co-glycolic acid), (50:50; 12000 mw), polyethylene glycol 300, polyethylene glycol 3350, polyethylene glycol 3350, polyethylene glycol 400, polyethylene glycol 4000, polyethylene glycol 600, polyglactin, polylactide, polyoxyethylene fatty acid esters, polyoxyl 15 hydroxystearate, polyoxyl 35 castor oil, polyoxyl 40 stearate, polypropylene, polysorbate 20, polysorbate 60, polysorbate 80, polyvinyl alcohol, potassium chloride, potassium hydroxide, potassium metabisulfite, potassium phosphate, monobasic, potassium sulfate, povidone k12, povidone/eicosene copolymer, povidones, proline, propylene glycol, propylene glycol monolaurate, propylparaben, protamine sulfate, protein hydrolysate, saccharin sodium, saccharin sodium anhydrous, serine, sesame oil, silastic medical adhesive, silicone type a, silicon, silicon dioxide, silicone, simethicone, sodium acetate, sodium acetate anhydrous, sodium ascorbate, sodium benzoate, sodium bicarbonate, sodium bisulfate, sodium carbonate, sodium carbonate monohydrate, sodium chloride, sodium formaldehyde sulfoxylate, sodium hydroxide, sodium iodide, sodium lactate, sodium lactate, I-, sodium lauryl sulfate, sodium metabisulfite, sodium n-(carbonyl-methoxypolyethylene glycol 2000)-1,2-distearoyl-sn-glycero-3-phosphoethanolamine, sodium oleate, sodium phosphate, sodium phosphate, dibasic, sodium phosphate, dibasic, anhydrous, sodium phosphate, dibasic, dihydrate, sodium phosphate, dibasic, dodecahydrate, sodium phosphate, dibasic, heptahydrate, sodium phosphate, monobasic, sodium phosphate, monobasic, anhydrous, sodium phosphate, monobasic, dihydrate, sodium pyrophosphate, sodium salicylate, sodium starch glycolate type a potato, sodium sulfate anhydrous, sodium sulfite, sodium tartrate, sodium tartrate dihydrate, sodium thiosulfate anhydrous, sorbic acid, sorbitan monolaurate, sorbitan monopalmitate, sorbitol, soybean oil, stannous chloride, stannous fluoride, stannous tartrate, stearic acid, stearic hydrazide, stearyl alcohol, succinic acid, sucrose, sucrose palmitate, sucrose stearate, sulfur, sulfuric acid, tagatose, tartaric acid, tartaric acid, dl-, tert-butyl alcohol, thimerosal, titanium dioxide, tocopherol, triacetin, tricaprilin, triethyl citrate, trihydroxystearin, trisodium citrate dihydrate, trisodium citrate dihydrate, trolamine, tromethamine, tryptophan, tyloxapol, undecylenic acid, urea, valine, vanillin, vegetable oil, vegetable oil, hydrogenated, versetamide, white wax, xylitol, yellow wax, zinc, zinc chloride, zinc oxide, 1,2-dimyristoyl-sn-glycero-3-phosphocholine, c13-14 isoparaffin/laureth-7/polyacrylamide, carbomer 1382, copovidone k25-31, laureth-23, polyethylene glycol 300, polyethylene glycol 3350, polyoxyl 35 castor oil, polyoxyl 6 and polyoxyl 32 palmitostearate, povidone k30, sodium n-(carbonyl-methoxypolyethylene glycol 2000)-1,2-distearoyl-sn-glycero-3-phosphoethanolamine, xylitol 300.

Preferably the liposome or the formulation is for medical use, preferably for use in the treatment of cancer.

Preferably the cancer is a solid tumor or a hematological malignancy, preferably the tumor is a triple negative breast tumor.

Preferably the tumor is selected from the group consisting of: Acute Lymphoblastic Leukemia (ALL), Acute Myeloid Leukemia (AML), Adrenocortical Carcinoma, Childhood Adrenocortical Carcinoma - see Unusual Cancers of Childhood, AIDS-Related Cancers, Kaposi Sarcoma (Soft Tissue Sarcoma), AIDS-Related Lymphoma (Lymphoma), Primary CNS Lymphoma (Lymphoma), Anal Cancer, Appendix Cancer - see Gastrointestinal Carcinoid Tumors, Astrocytomas, Childhood (Brain Cancer), Atypical Teratoid/Rhabdoid Tumor, Childhood, Central Nervous System (Brain Cancer), Basal Cell Carcinoma of the Skin, Skin Cancer, Bile Duct Cancer, Bladder Cancer, Childhood Bladder Cancer, Bone Cancer (includes Ewing Sarcoma and Osteosarcoma and Malignant Fibrous Histiocytoma), Brain Tumors, Breast Cancer, Childhood Breast Cancer, Bronchial Tumors, Childhood, Burkitt Lymphoma, Non-Hodgkin Lymphoma, Carcinoid Tumor (Gastrointestinal), Carcinoid Tumor (Lung and other sites), Childhood Carcinoid Tumors, Carcinoma of Unknown Primary, Childhood Carcinoma of Unknown Primary, Cardiac (Heart) Tumors, Childhood, Central Nervous System, Atypical Teratoid/Rhabdoid Tumor, Childhood (Brain Cancer), Embryonal Tumors, Childhood (Brain Cancer), Germ Cell Tumor, Childhood (Brain Cancer), Primary CNS Lymphoma, Cervical Cancer, Childhood Cervical Cancer, Cholangiocarcinoma, Chordoma, Childhood, Chronic Lymphocytic Leukemia (CLL), Chronic Myelogenous Leukemia (CML), Chronic Myeloproliferative Neoplasms, Colorectal Cancer, Childhood Colorectal Cancer, Craniopharyngioma, Childhood (Brain Cancer), Cutaneous T-Cell Lymphoma, Lymphoma (Mycosis Fungoides and Sezary Syndrome), Ductal Carcinoma In Situ (DCIS), Breast Cancer, Embryonal Tumors, Central Nervous System, Childhood (Brain Cancer), Endometrial Cancer (Uterine Cancer), Ependymoma, Childhood (Brain Cancer), Esophageal Cancer, Childhood Esophageal Cancer, Esthesioneuroblastoma (Head and Neck Cancer), Ewing Sarcoma (Bone Cancer), Extracranial Germ Cell Tumor, Childhood, Extragonadal Germ Cell Tumor, Eye Cancer, Childhood Intraocular Melanoma, Intraocular Melanoma, Retinoblastoma, Fallopian Tube Cancer, Fibrous Histiocytoma of Bone, Malignant, and Osteosarcoma, Gallbladder Cancer, Gastric (Stomach) Cancer, Childhood Gastric (Stomach) Cancer, Gastrointestinal Carcinoid Tumor, Gastrointestinal Stromal Tumors (GIST) (Soft Tissue Sarcoma), Childhood Gastrointestinal Stromal Tumors, Germ Cell Tumors, Childhood Central Nervous System Germ Cell Tumors (Brain Cancer), Childhood Extracranial Germ Cell Tumors, Extragonadal Germ Cell Tumors, Ovarian Germ Cell Tumors, Testicular Cancer, Gestational Trophoblastic Disease, Gliomas, Hairy Cell Leukemia, Head and Neck Cancer, Heart Tumors, Childhood, Hepatocellular (Liver) Cancer, Histiocytosis, Langerhans Cell, Hodgkin Lymphoma, Hypopharyngeal Cancer (Head and Neck Cancer), Intraocular Melanoma, Childhood Intraocular Melanoma, Islet Cell Tumors, Pancreatic Neuroendocrine Tumors, Kaposi Sarcoma (Soft Tissue Sarcoma), Kidney (Renal Cell) Cancer, Langerhans Cell Histiocytosis, Laryngeal Cancer (Head and Neck Cancer), Leukemia, Lip and Oral Cavity Cancer (Head and Neck Cancer), Liver Cancer, Lung Cancer (Non-Small Cell and Small Cell), Childhood Lung Cancer, Lymphoma, Male Breast Cancer, Malignant Fibrous Histiocytoma of Bone and Osteosarcoma, Melanoma, Childhood Melanoma, Melanoma, Intraocular (Eye), Childhood Intraocular Melanoma, Meningiomas, Merkel Cell Carcinoma (Skin Cancer), Mesothelioma, Malignant, Childhood Mesothelioma, Metastatic Cancer, Metastatic Squamous Neck Cancer with Occult Primary (Head and Neck Cancer), Midline Tract Carcinoma With NUT Gene Changes, Mouth Cancer (Head and Neck Cancer), Multiple Endocrine Neoplasia Syndromes, Multiple Myeloma/Plasma Cell Neoplasms, Mycosis Fungoides (Lymphoma), Myelodysplastic Syndromes, Myelodysplastic/Myeloproliferative Neoplasms, Myelogenous Leukemia, Chronic (CML), Myeloid Leukemia, Acute (AML), Myeloproliferative Neoplasms, Chronic, Nasal Cavity and Paranasal Sinus Cancer (Head and Neck Cancer), Nasopharyngeal Cancer (Head and Neck Cancer), Neuroblastoma, Non-Hodgkin Lymphoma, Non-Small Cell Lung Cancer, Oral Cancer, Lip and Oral Cavity Cancer and Oropharyngeal Cancer (Head and Neck Cancer), Osteosarcoma and Malignant Fibrous Histiocytoma of Bone, Ovarian Cancer, Childhood Ovarian Cancer, Pancreatic Cancer, Childhood Pancreatic Cancer, Pancreatic Neuroendocrine Tumors (Islet Cell Tumors), Papillomatosis (Childhood Laryngeal), Paraganglioma, Childhood Paraganglioma, Paranasal Sinus and Nasal Cavity Cancer (Head and Neck Cancer), Parathyroid Cancer, Penile Cancer, Pharyngeal Cancer (Head and Neck Cancer), Pheochromocytoma, Childhood Pheochromocytoma, Pituitary Tumor, Plasma Cell Neoplasm/Multiple Myeloma, Pleuropulmonary Blastoma, Pregnancy and Breast Cancer, Primary Central Nervous System (CNS) Lymphoma, Primary Peritoneal Cancer, Prostate Cancer, Rectal Cancer, Recurrent Cancer, Renal Cell (Kidney) Cancer, Retinoblastoma, Rhabdomyosarcoma, Childhood (Soft Tissue Sarcoma), Salivary Gland Cancer (Head and Neck Cancer), Sarcoma, Childhood Rhabdomyosarcoma (Soft Tissue Sarcoma), Childhood Vascular Tumors (Soft Tissue Sarcoma), Ewing Sarcoma (Bone Cancer), Kaposi Sarcoma (Soft Tissue Sarcoma), Osteosarcoma (Bone Cancer), Soft Tissue Sarcoma, Uterine Sarcoma, Sezary Syndrome (Lymphoma), Skin Cancer, Childhood Skin Cancer, Small Cell Lung Cancer, Small Intestine Cancer, Soft Tissue Sarcoma, Squamous Cell Carcinoma of the Skin, Squamous Neck Cancer with Occult Primary, Metastatic (Head and Neck Cancer), Stomach (Gastric) Cancer, Childhood Stomach (Gastric) Cancer, T-Cell Lymphoma, Cutaneous, Lymphoma (Mycosis Fungoides and Sezary Syndrome), Testicular Cancer, Childhood Testicular Cancer, Throat Cancer (Head and Neck Cancer), Nasopharyngeal Cancer, Oropharyngeal Cancer, Hypopharyngeal Cancer, Thymoma and Thymic Carcinoma, Thyroid Cancer, Transitional Cell Cancer of the Renal Pelvis and Ureter (Kidney (Renal Cell) Cancer), Unknown Primary, Carcinoma of, Childhood Cancer of Unknown Primary, Unusual Cancers of Childhood, Ureter and Renal Pelvis, Transitional Cell Cancer (Kidney (Renal Cell) Cancer, Urethral Cancer, Uterine Cancer, Endometrial, Uterine Sarcoma, Vaginal Cancer, Childhood Vaginal Cancer, Vascular Tumors (Soft Tissue Sarcoma), Vulvar Cancer, Wilms Tumor and Other Childhood Kidney Tumors, Preferably the cancer is in adolescents, young adults, elderly, adults, infants, new borns, children.

The liposome or formulations of the invention may be administered by any route which effectively transport the liposomes to the appropriate or desirable site of action. Preferred modes of administration include intravenous (IV) and intra-arterial (IA). Other suitable modes of administration include intramuscular (IM), subcutaneous (SC), intranasal, and intraperitonal (IP). Such administration may be bolus injections or infusions. Another mode of administration may be by perivascular delivery. The formulation may be administered directly or after dilution. Combinations of any of the above routes of administration may also be used in accordance with the invention. Any route of administration may be utilized provided that the particles are in contact with phagocytic cells (e.g., circulating monocytes or peritoneal macrophages).

The formulation for use in accordance with the present invention may be formulated using one or more physiologically acceptable carriers comprising excipients and auxiliaries known in the art, which facilitate processing of the active ingredients into preparations which, can be used pharmaceutically.

Preferably the liposome or formulation of the invention is used in a therapeutically effective amount.

As used herein, the phrase "therapeutically effective amount" shall mean the drug dosage that provides the specific pharmacological response for which the drug is administered in a significant number of subjects in need of such treatment. The actual effective amounts of compound or drug can vary according to the specific composition being utilized, the mode of administration and the age, weight and condition of the patient. For example, as used herein, an effective amount of the drug is an amount which prevent or treat or slow down the progression of a tumor. Dosages for a particular individual patient can be determined by one of ordinary skill in the art using conventional considerations, (e.g. by means of an appropriate, conventional pharmacological protocol).

The present invention also provides a method of production of a liposome as defined above comprising the steps of:
- combining a a poly(ethylene glycol)-lipid (PEG-lipid) or a derivative thereof with cholesterol to obtain a lipid suspension;
- adding an anti-LOX antibody to said lipid suspension and
- adding a therapeutic agent.

The present invention will be illustrated by means of non-limiting examples in reference to the following figures.

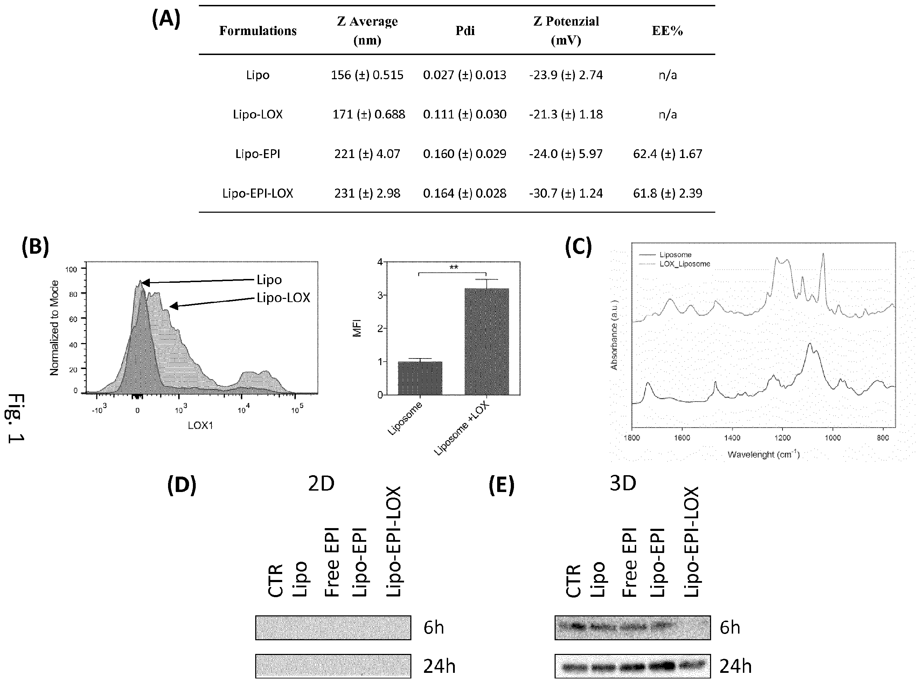

Figure 1. (A) Physical characterization of lipid-based nanocarriers average diameter (Z AVERAGE), polydispersivity index (PDI), Z Potential (mV) and encapsulation efficiency percentage (EE%). (B) Cytometry analysis of Lipo and Lipo-LOX formulations mean florescence intensity (MFI), p<0.001 (**) (C) FTIR analysis of Lipo and Lipo-LOX formulations. (D) Protein analysis of secreted LOX in monolayer cultures not treated and treated with all formulations: control (CTR), empty liposomes (Lipo), epirubicin (Free Epi) liposomes incapsulated with epirubicin (Lipo-EPI) and liposomes encapsulated with epirubicin and decorated with anti-LOX (Lipo-EPI-LOX). (E) Protein analysis of secreted LOX in 3D cultures not treated and treated with all formulations.

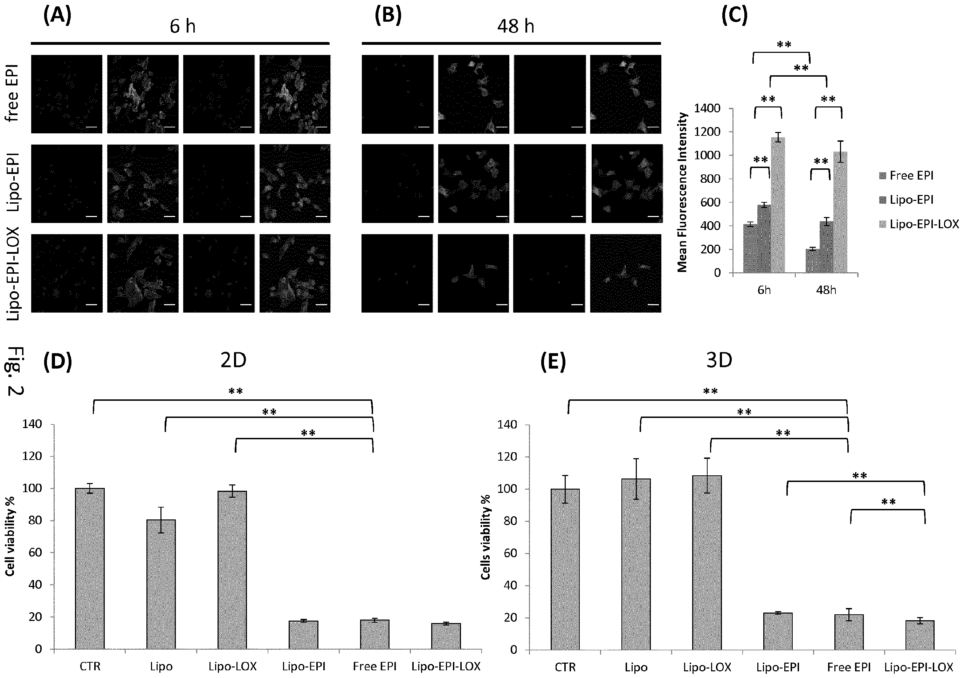

Figure 2. (A) Confocal analysis of 3D cultures exposed to formulations encapsulated with anthracycline after 6 h from the exposure. (B) Confocal analysis of 3D cultures exposed to formulations encapsulated with anthracycline after 48 h from the exposure. Scale bar 50 µm (C) Mean fluorescence intensity of anthracycline detected after 6 and 48 h. (D) Cell viability of tumor cells after treatment with all studied formulations in standard monolayer cultures. (E) Cell viability of tumor cells after treatment with all studied formulations in 3D cultures. * *p* < 0.05, ***p* < 0.01.

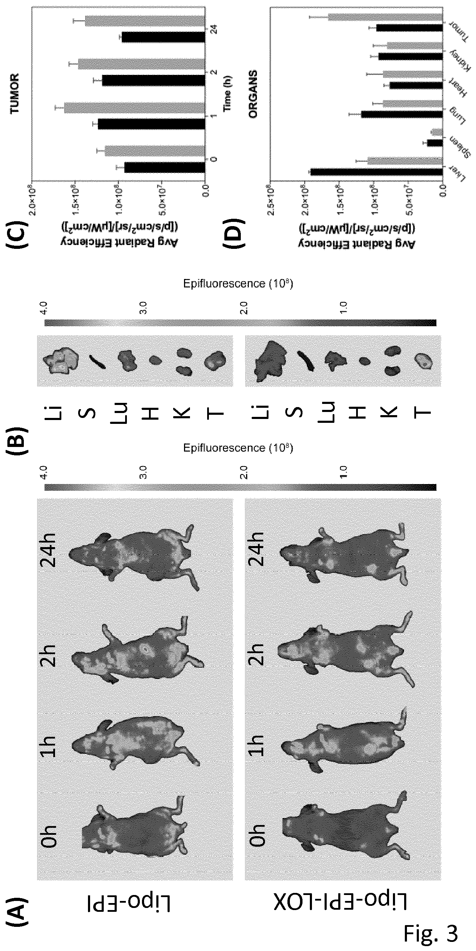

Figure 3. (A) In vivo biodistribution analysis of Lipo-EPI and Lipo-EPI-LOX in orthotropic xenograft mouse model of human TNBC at different time points (0, 1, 2 and 24 h post injections) (B) bioluminescence and fluorescence analysis of explanted organs (liver LI, spleen S, lungs Lu, heart H, kidneys K, tumors T) to detect EPI localization. (C) Lipo-EPI (black bars) and Lipo-EPI-LOX (grey bars) fluorescent signal quantification in tumor. (D) Lipo-EPI (black bars) and Lipo-EPI-LOX (grey bars) fluorescent signal quantification in explanted organs at 24 h.

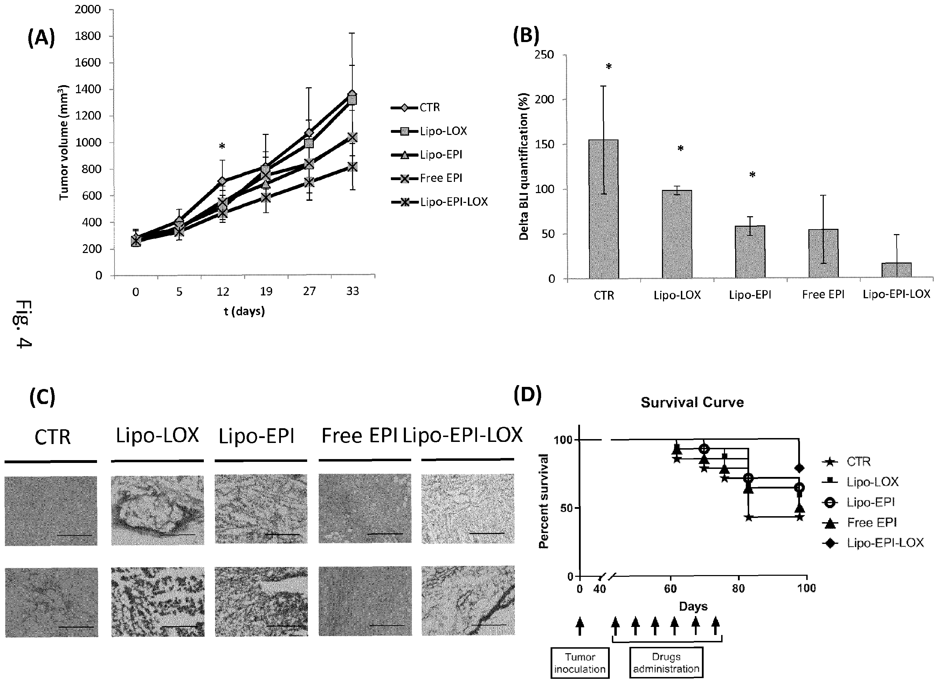

Figure 4. (A) Tumor growth curves of orthotropic xenograft mouse model of human TNBC. Five group mice (n=10 each group) treated with empty liposome, Lipo-LOX, LIPO-EPI, free EPI and Lipo-EPI-LOX. * *p* < 0.05. (B) Delta quantification of TNBC bioluminescent signal after 4 weeks of treatment. * *p* < 0.05. (C) Representative example of H&E stained sections of explanted tumors 55 days post treatment. Control: markedly atypical and pleomorphic cells (5% necrotic tumor cells). Scale bar is 400 µm. Lipo-LOX: fibrotic tissue with atipic cells and neoplastic crown (25% necrotic tumor cells). Lipo-EPI: Neoplastic cells and necrotic bands (50% necrotic tumor cells). Free EPI: tumor cells infiltrating adipose tissue with minimal necrotic features and limited inflammation (20% necrotic tumor cells). Lipo-EPI-LOX : tumor cells infiltrating adipose tissue with artifacts (60% necrotic tumor cells). (D) Kaplan-Meier curve indicating survival in tumor-bearing mice after tumor induction in the listed treatment groups (Control, Lipo-LOX, LIPO-EPI, free EPI and Lipo-EPI-LOX).

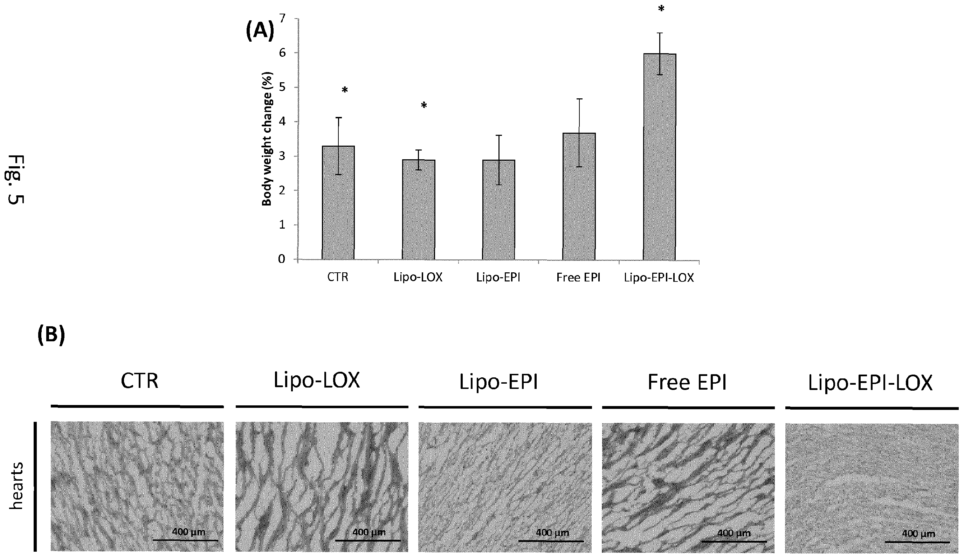

Figure 5. (A) body weight change percentage of mice at the end of the study (98 days after tumor cells inhoculation) * *p* < 0.05 (B) Representative example of H&E stained sections of explanted hearts 55 days post treatment. Control: normal cardiac tissue. Lipo-LOX: no increase in inflammatory infiltrate. Lipo-EPI: minimal focal increase of inflammatory infiltrate (granulocytes and lymphocytes), artifacts. Free EPI: increased cellularity due to possible inflammatory infiltrate. Lipo-EPI-LOX: no significant increment in lymphoid infiltrate, artifacts.

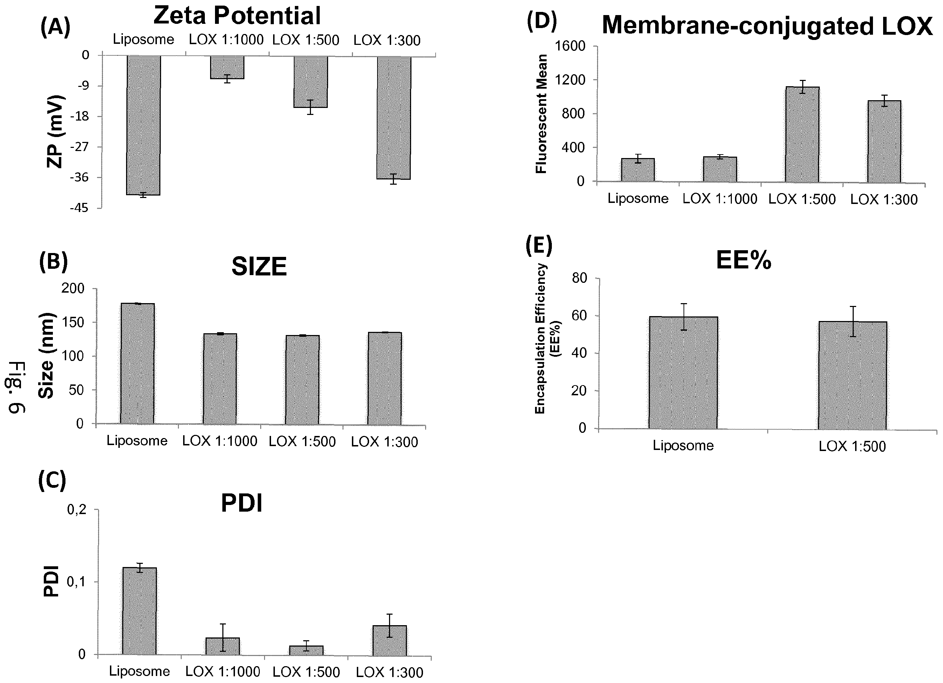

Figure 6. Physicochemical characterization of PEGylated liposomes (liposomes) and LOX-conjugated PEGylated liposomes (LOX). Three different LOX-to-lipid molar ratios (1:1000, 1:500, and 1:300) were tested. The presence of the LOX on carrier's surface affected the surface charge, causing a significant reduction of the zeta potential (ZP) values (A). DLS analysis shows how the conjugation of LOX on the surface of liposomes brought to a collapse of the vesicular structure, as can be noted by the reduction of vesicle diameter (B), as well as a stabilization of the formulation, as indicated by the reduction of the polidispersivity index (PDI) (C). Next, flow cytometry analysis was performed in order to evaluate the best LOX-to-lipid ratio in terms of LOX density on liposome surface (D). The analysis revealed that 1:500 LOX-to-lipid ratio permitted to obtain the highest density of LOX on the surface compared to 1:1000 and 1:300. Lastly, the encapsulation of epirubicin using the remote loading method revealed a loading of about 60% encapsulation efficiency percentage (EE%) (E). Most importantly, compared to liposomes, the presence of LOX on the surface did not significantly affect the amount of drug encapsulated.

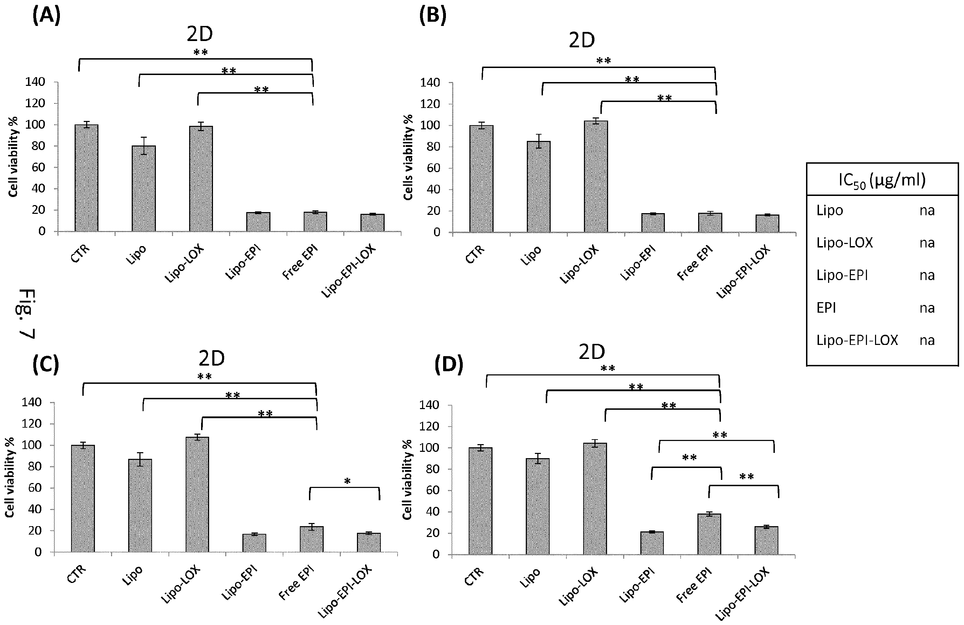

Figure 7. Cell viability of tumor cells after treatment with all studied formulations in standard monolayer cultures. (A) Cell viability using formulations with epirubicin plasma peak concentration (B) Cell viability using formulations with epirubicin half-plasma peak concentration (C) Cell viability using formulations with epirubicin one-quarter plasma peak concentration (D) Cell viability using formulations with epirubicin one-eight plasma peak concentration * *p* < 0.05, ***p* < 0.01. Not applicable (na).

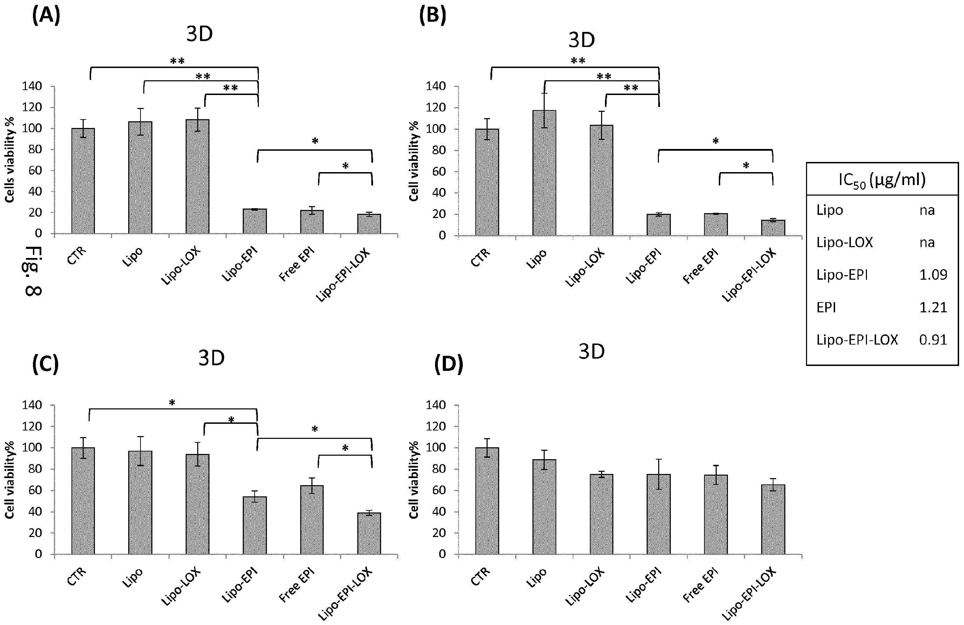

Figure 8. Cell viability of tumor cells after treatment with all studied formulations in 3D cultures. (A) Cell viability using formulations with epirubicin plasma peak concentration (B) Cell viability using formulations with epirubicin half-plasma peak concentration (C) Cell viability using formulations with epirubicin one-quarter plasma peak concentration (D) Cell viability using formulations with epirubicin one-eight plasma peak concentration * p < 0.05, **p < 0.01.

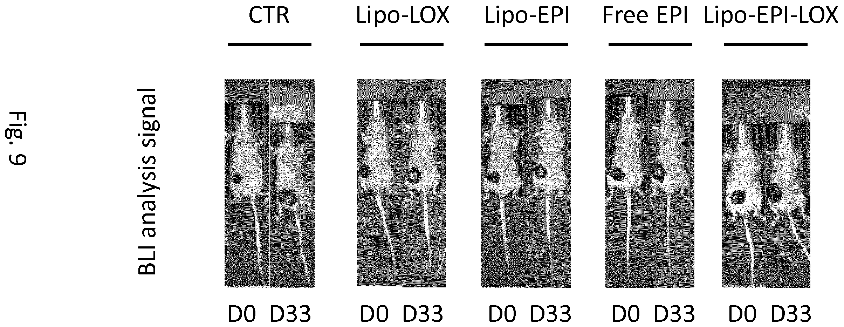

Figure 9. Bioluminescence signal of TNBC in all treatment at starting treatments (D0) and after 5 weeks of treatments (D33).

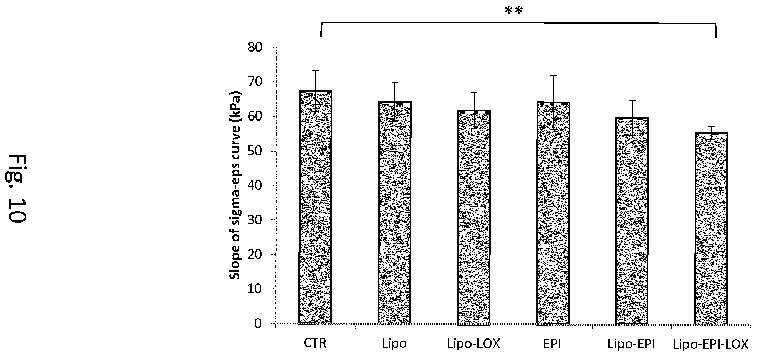

Figure 10. Stiffness values for CTR, Lipo, Lipo-LOX, Lipo-EPI, Free EPI, Lipo-EPI-LOX scaffolds after 72 h of treatment, expressed as the slope of the sigma-epsilon curve (sigma-eps) (kPa).

### DETAILED DESCRIPTION OF THE INVENTION

### EXPERIMENTAL METHODS

### Fabrication and physical characterization of LOX engineered lipid vesicles

Briefly, lipid vesicles were prepared by the well-established thin-layer evaporation (TLE) procedure. 1,2-distearoyl-sn-glycero-3-phosphocholine (DSPC), cholesterol, DSPE-PEG(2000) and DSPE-PEG(2000)-succynil (catalog number 850365, 700001, 880229, 880121 Avanti Polar Lipid) were dissolved in chloroform with a final lipid concentration of 20 mg/mL. The molar ratio of DSPC/cholesterol/DSPE-PEG(2000)/DSPE-PEG(2000)-succynil was 60:30:5:5. The solvent was evaporated through a rotary evaporator (Buchi Labortechnik AG, Switzerland) for 20 min at 45°C to form a thin lipid film. Film was hydrated with sterile saline solution to assemble lipid vesicles (CTR). Then the solution was heated at 75°C and mixed with a vortex three times. Lipid suspension was forced ten times through 2 polycarbonate filters (100 nm; GE Osmonics Labstore, Minnetonka, MN) under nitrogen gas pressure at 45°C. Anti-LOX (Abcam Cambridge UK, ab31238, MW 36 KDa, 100 mg/mL), was then added to this mixture, and the suspension was incubated overnight at 4°C with gentle stirring (with different anti-LOX/lipid molar ratio, 1:1000, 1:500, 1:300). Liposomes purification was performed by 1 h dialysis through 1000 KDa membranes (Spectrum Laboratories, Inc.) and then the samples were stored at 4°C. Physical characterization was carried out with a Nanosizer ZS (Malvern Instruments, Malvern, Worcestershire, UK). LOX conjugation was validated by flow cytometry analysis. Briefly, a secondary antibody (FITC-labeled, Alexa fluo 488) was incubated with liposomes and LOX-functionalized liposomes in MES buffer solution (1:1000 dilution, pH 7.5) for 1 h at room temperature. After 1 h dialysis through 1000 KDa membranes, FITC signal was detected by flow cytometry.

### Drug loading

Briefly, liposomes were prepared by the thin-film method. DSPC, cholesterol (Chol), DSPE-PEG(2000) and DSPE-PEG(2000)-succynil were combined in chloroform with a final lipid concentration of 20 mg/mL. The molar ratio of DSPC/CHOL/DSPE-PEG(2000)/DSPE-PEG(2000)-succynil was 60:30:5:5. Once the lipids were thoroughly mixed in the chloroform, the chloroform was evaporated with a rotavapor for 20 min at 45°C to form a thin lipid film. 1 ml of ammonium sulfate was then added to the thin film. Then the solution was heated at 68°C and mixed with a vortex three times (heat and vortex 3 times). Then the lipid suspension was forced ten times through 2 polycarbonate filters (100 nm; GE Osmonics Labstore) under nitrogen gas pressure at 75°C (extruder). Then the solution was centrifuged at 36000 rpm (14000 g) in a ultracentrifuge with a eppendorf tube for 1 h at 4°C. Then the pellet was resuspended in 2.5 ml of epirubicin solution (5mg/ml epirubicin Ellence, epirubicin hydrocloride, Pfizer, New York, USA, NDC 0009-5091-01). Then the solution was heated with a termomixer at 60°C for 2 h then centrifuged to eliminate the unloaded drug and stored at 4°C.

### Preparation of Lipo and Lipo-LOX formulation

### example: 1 preparation

- DSPC 10.8 mg
- CHOL 2.7 mg
- DSPE-PEG 3.2 mg
- DSPE-PEG-succynil 3.3 mg

### example: 6 preparations

- DSPC 64.8 mg
- CHOL 16.2 mg
- DSPE-PEG 19.2 mg
- DSPE-PEG-succynil 19.8 mg
   - All the lipids are mixed in 1 ballon with 6 ml of chlorophorm. Then the mixture is splitted in 6 ballons, and another 1 ml of chlorophorm is added to each ballon
   - rotavapor 45°C 20 min (then see drug loading section for the specific preparations)
   - add 1 ml MilliQ water and 3 min at 45°C and 3 min vortex for 3 times
   - 10 extrusions, collect each preparation in a 15 ml falcon tube
   - ab conjugation overnight
   - Liposomes purification 1 h dialysis through 1000 KDa membranes (all membranes in a beacker with 1 liter of water and stirring)
   - for facs analysis put 600 ul of preparation in a eppendorf and add 0.6ul of Alexa fluo 488 anti-LOX

### Preparation of Lipo-EPI and Lipo_EPI-LOX formulations

- for drug loading put in the ballon 1 ml of ammonium sulfate 250 milli Molar (33 mg/ml)
- incubate at 68°C 3 min vortex 3 min for 3 times
- 10 extrusions, collect each preparation in a 15 ml falcon tube
- put 1 ml of preparation in an eppendorf tube
- centrifuge 36000 rpm (14000 g) 1 h 4°C
- resuspend the pellet in 0,5 ml of epirubicin solution
- thermomixer 60°C for 2 h
- centrifuge
- ready to use

### Physical characterization of lipid-based nanocarriers

Average diameter (Z AVERAGE), polydispersivity index (PDI), Z Potential (mV) and encapsulation efficiency percentage (EE%) were obtained as reported in [24] using a Nanosizer ZS (Malvern Instruments, Malvern, Worcestershire, UK as reported in 24.

### Cytometry analysis

For LOX surface expression, LOX-functionalized liposomes were prepared as above described. Briefly, particles were added to 1% bovine serum albumin in phosphate buffered saline solution with a final concentration of 0.5 mM. Next, anti-LOX antibody (Abcam) was added at 2.5 µg/mL and allowed to incubate for 30 min. at 4 °C. Following incubation, particles were dialyzed using a Float-A-Lyzer G2 dialysis device (Spectrum Labs) with a 1000 kDa dialysis membrane for 1 h in Milli-Q water to remove unbound antibody. Following dialysis, particles were incubated with an AlexaFluor 488-conjugated secondary antibody (goat anti-mouse IgG, Abcam) for 1 h at 4 °C and immediately followed by dialysis as previously described. Flow Cytometry was performed using a BD LSRFortessa cell analyzer with further analysis conducted using FlowJo X.

In order to further evaluate liposomes anti-LOX coupling Fourier Transform Infrared spectroscopy (FTIR) analysis in attenuated total reflection using a single reflection diamond element were performed. For the study, the FTIR spectrometer Nicolet was used. Lipid vesicles drug loading (Lipo-EPI and Lipo-EPI-LOX) was obtained through passive loading techniques. Lipid films were hydrated with a epirubicin hydrochloride solution (Ellence, epirubicin hydrocloride, Pfizer, New York, USA, NDC 0009-5091-01) (epirubicin/lipid molar ratio 1:4). For drug encapsulation efficiency percentage, the concentration of epirubicin was determined in the lysed liposomes and in the supernatant at 495 nm using a plate reader (BioTek, Synergy H4 hybrid reader) and analyzed based on a standard curve for epirubicin.

### Fourier Transform Infrared spectroscopy (FTIR) measurements

Both Liposome and Lipo-LOX have been suspended at the concentration of 1 mg/ml in PBS. Fourier Transform Infrared spectroscopy was employed for the particles characterization, since it enable to detect the proteic part over the lipidic structure of the liposomes. Fourier Transform Infrared spectroscopy measurements in attenuated total reflection (ATR) were performed using a single reflection diamond element. The FTIR spectrometer Nicolet was employed under the following conditions: 2 cm⁻¹ spectral resolution, 20 kHz scan speed, 1000 scan co-addition, and triangular apodization. 5 µl of each sample were deposited on the ATR plate and spectra were recorded after solvent evaporation to allow the formation of a hydrated lipophilic film. 5 ul of the buffer has been recorder to use as background. The ATR/FTIR spectra were reported after binomial smoothing (11 points) and the subtraction of background.

### Theoretical calculations of lipo-LOX surface coupling

For anti-LOX quantification a solution of 1 µg/ml of liposomes decorated conjugated with anti-LOX was incubated with 2 µg/ml of LOX secondary antibody. Florescence obtained from Lipo or Lipo-EPI were subtracted as background. The results were interpolated in a LOX calibration curve previously prepared. Moreover, an indirect quantification of anti-LOX coupling to liposomes was performed with the analysis of supernatants. Finally, to corroborate the results previously obtained the Bradford micro assay (Quick Start Bradford Protein assay, Bio Rad Laboratories, Los Angeles, CA) was used to quantify the amount of antibody conjugated to the liposome. A spectrophotometer was used to analyze the samples at 595 nm. The quantification was performed using a standard calibration curve obtained using bovine serum albumin. Anti-LOX concentration was expressed as the average between fluorescence and protein analysis results and converted to µg of antibody per 100 µg of polymer liposomes.

### Cells seeding and culturing

All the study was performed with the use of human breast cancer cell line MDA-MB-231 purchased from the American Type Culture Collection (HTB-26™ Rockville, Maryland, USA). The cells were maintained in Dulbecco's modified Eagle's medium (DMEM) supplemented with 10% fetal bovine serum, 1% penicillin-streptomycin and 1% glutamine (ECB7501L, ECS0180D, ECB3001D, ECB3000D-20 Milan, Italy) at 37°C in a 5% CO₂ atmosphere. For standard cultures, 2.5x10⁶ cells were seeded and maintained as a monolayer in 75-cm² flasks. Passages were performed according to manufacturer's instruction. For tridimensional cultures, 5x10⁶ cells were cultured for 7 days in scaffolds, the medium was replaced daily [27]. All the experiments were conducted using low-passage (under 30) MDA-MB-231 cell line and in active proliferation (reach 90% of confluency every 2-3 days).

### Validation of lipo-LOX activity

In order to confirm the activity of LOX antibody after the liposomes functionalization, secreted LOX protein was quantified in the cells culture medium at different time point. Briefly, MDA-MB-231 cultured both in standard monolayer and tridimensional culture were exposed to liposomes (Lipo), epirubicin (Free EPI), epirubicin-loaded liposomes (Lipo-EPI), and epirubicin-loaded anti-LOX liposomes (Lipo-EPI-LOX) at the concentration of the human plasma peak as specified in the drug testing section. Culture medium was collected at 6, 24 and 48 hours respectively. The medium was centrifuged at 15,000 rpm for 20 min at 4°C and the pellet was discarded in order to eliminate cell debris. The protein contents were determined using a BCA protein assay kit (Pierce™ BCA Protein Assay Kit, Thermo Scientific). An equal amount of protein from each sample was separated on Criterion™ Precast Gel Tris-HCI (Biorad, Hercules, CA, USA) and transferred to polyvinylidene fluoride membranes (Millipore Corporation). The membranes were blocked for 2 h with a solution containing 5% fat-free milk PBS with 0.1% Tween 20 (Sigma-Aldrich) at room temperature, and incubated overnight at 4°C with anti-LOX antibody (1:1000 CAT # ab31238, Abcam, Cambridge, UK). The membranes were then washed and incubated for 1 h at room temperature with horseradish-peroxidase-conjugated secondary antibody.

### Microscopy analysis

In order to confirm the EPI delivery ability of Lipo-LOX formulation confocal analysis was performed on 3D culture. The culture was exposed to Lipo-EPI and Lipo-EPI-LOX for 72 h, then the cells where washed 3 times with 1% PBS, fixed with 4% paraformaldehyde for 20 minutes at room temperature and stained for for DAPI (1:1000, Life Technologies, Carlsbad, CA, USA) and Phalloidin (1:40 Alexa Fluor 488 phalloidin, Life Technologies, Carlsbad, CA, USA). Images where acquired with an A1 laser confocal microscope (Nikon Corporation, Tokyo, Japan), and analyzed with the NIS-Elements software (Nikon Corporation, Tokyo, Japan).

### In vitro drug testing

Cells human breast cancer cell line MDA-MB-231 (ATCC® HTB-26™, American Type Culture Collection (ATCC), Manassas, USA) were cultured in monolayer cultures or in 3D scaffolds for 24 h before exposure to the drugs. Drug regimens were selected according to the plasma peak concentration of epirubicin from pharmacokinetic clinical data, 3.4 µg/ml [28] (which correspond to 110 µg of lipid component for lipo-EPI and lipo-EPI-LOX per ml). For lipo-LOX treatment group was 0,44 ug of anti-LOX which correspond to the same concentration of theoretical LOX used for the lipo-EPI-LOX (0,11 µg of anti-LOX per 4 ml).

For lipo treatment group was used the same concentration of lipid vesicles used for lipo-LOX (110 µg/ml). Cell viability percentage was assessed by 3-(4,5-Dimethylthiazol-2-yl)-2,5-Diphenyltetrazolium Bromide (MMT) assay (MFCD00080731 Sigma Aldrich, St. Louis, MO, USA) after a 72-hour drug exposure. The IC₅₀ values were calculated from the non-linear regression of the dose-log response curves with GraphPad Prism 8. Experiments were performed in triplicate.

### In vivo study

All animal experiments were performed in accordance with the guidelines of the Animal Welfare Act and the Guide for the Care and Use of Laboratory Animals approved by the Institutional Animal Care and Use Committee (IACUC) of the Houston Methodist Research Institute (HMRI) protocol number AUP-0614-0033.

### Orthotopic xenograft

MDA-MB-231 labeled with luciferase probe (2x10⁶ cells/100 µl matrigel) were orthotopically injected into the right mammary fad pad of female immunodeficient NU/NU nude mice [NU-Foxn1nu] 8-10 weeks old (Charles River Laboratories, Wilmington, MA, USA). NU/NU nude mice were housed five per cage at the HMRI animal facility, there were maintained under pathogen free conditions and on low-fluorescence diet according to the National Institutes of Health guidelines.

### Tumor growth and body weight measurements

Tumor size and body weight were measured with caliper and a digital balance once a week. Tumor volume was calculated according to the formula (*W x W x L*)/2, where *W* and *L* represent the width and the length of tumor, respectively. Body weight change % was calculated were calculated by comparing weights at the start and end of therapy throught the formula throught the formula weight D0/weight D33 %.

Furthermore, the impact on tumor growth was validated by performing bioluminescence imaging (BLI) of TNBC tumors at Day 0 and Day 33 (Figure 4B, Figure S4 supportive information). This was performed in order to support the caliper measurements since the bioluminescent signal obtained from BLI is directly related to the viability of cells and could serve as metric to evaluate impact of therapy. The BLI quantification percentage was calculated throught the formula BLI D0/BLI D33 %.

### In vivo drug testing

Once the tumors reached a 270 mm³ median volume NU/NU nude mice were sorted into groups for treatment with anti-LOX liposomes (Lipo-LOX), epirubicin-loaded liposomes (Lipo-EPI), epirubicin (free EPI), epirubicin-loaded anti-LOX liposomes (Lipo-EPI-LOX) and control (CTR empty liposomes). Mice bearing human breast cancer xenografts received weekly i.v. injections of drugs 3,24 mg/Kg (n=10 per cohort) for five weeks. Dosages were selected according to the human plasma peak of epirubicin from pharmacokinetic clinical data and converted to mice equivalent surface area [29]. For Lipo-LOX group and equivalent concentration of liposomes used for the above groups was administered.

### Survival study

Mice (n=10 per cohort) were sacrificed when the tumor volume reached 2000 mm³ or when showing signs of morbidity. Data were analyzed using the Kaplan-Meier method. Comparisons among different treatment groups were performed using the log rank test for trend and considered significant with a *p* value less than 0.05.

### Biodistribution of EPI in MDA-MB-231 tumors

Nu/Nu mice were implanted with MDA-MB-231 tumors as described above. When tumors reached a size of 260-280 mm³, mice were imaged for bioluminescence and fluorescence to collect background signal. Imaging was performed using an IVIS Spectrum housed within HMRI's Translation Imaging - PreClinical Imaging (Small Animal) Core, as previously described by us [30-32]. Here, the inventors used bioluminescence imaging to determine the location of tumors using the same procedure discussed above. Fluorescence imaging was done by collecting images using 500/620 (excitation/emission) filters to follow the distribution of EPI. After imaging background signal (t = 0h), mice were separated into groups for treatment with Lipo-EPI (n=2) or Lipo-EPI-LOX (n=3). At 1, 2, and 24 hours, mice were imaged for bioluminescence and fluorescence. Mice were sacrificed after 24 hours and their organs were imaged using the same settings. Images and quantification of EPI distribution were analyzed using Living Image 4.5, where bioluminescent images served to create tumor ROIs for each time point.

### Histology of organs and tumor tissues

Explanted mice organs (heart, liver, spleen, kidneys, lungs, tibia) and tumors were washed twice with PBS, embedded in a cryomold in O.C.T. (Tissue-Tek® O.C.T. Compound, Sakura® Finetek), and frozen at -80 degree. Ten µm-thick slides were obtained cutting tissues block with a cryostat at -20 degree. The slides were then stored at -20 degree until the analysis.

### Histological analysis

Hematoxylin and eosin (H&E) staining was performed to evaluate the efficacy and safety of Lipo-EPI-LOX compared to the other treatment groups. Briefly the organs and tumor slides were thawed, hydrated and stained with H&E following the manufacturer's instructions. The stained slides were analyzed with an optical Zeiss Axioskop microscope (Carl Zeiss, Gottïngen, Germany) equipped with a Polaroid camera.

### Collagen quantification

Tumors frozen 5-µm-thick slides were fixed for 5 min in formalin 10% solution, then washed and stained with Weigert's haematoxylin. Then the sections were stained with 0.1% (Direct Red 80) (Sigma Aldrich) picrosirius red in saturated aqueous solution of picric acid. The stained slides were analyzed with the NIS Elements software (Nikon Corporation, Tokyo, Japan).

### Mechanical Testing

As previously described [33], an in-house built instrument prototype was developed by the inventors to apply a state of unconfined uniaxial compression to the collagen scaffolds. The applied stress (r, in kPa) could then be analyzed by dividing the applied force by the cross section of the sample. The applied force consisted of a pre-load of 1.41 kPa held constantly for 30 s; the full load of 5.97 kPa was then constantly applied for 30 s. The stiffness (compressive modulus, in kPa) of the collagen scaffold cultured with MDA-MB-231 and exposed for 72h to all treatment groups was computed as the ratio between the increment of stress and increment of strain from the preload to the full load values. The samples were analyzed in air immediately after having been removed from the PBS where they were soaked. Five samples of each type were tested and each specimen was tested 10 times.

### Statistical analysis

Three independent replicates were performed for each experiment. Data are presented as mean ± SD, or mean ± SE, as stated, with n indicating the number of replicates. For in vitro and in vivo data, differences between groups were assessed by a two-tailed Student's t-test or analysis of variance (ANOVA) and accepted as significant at p < 0.05. For EPI biodistribution studies, differences in EPI accumulation in tumors at various times and in organs was assessed using multiple t tests (one unpaired t-test per comparison) assuming samples with same scatter and accepted as significant at p < 0.05.

### EXAMPLES

### Example 1: Engineered-anti-LOX liposomes designing study

The inventors fabricated biocompatible PEGylated liposomes [34] using the well-established thin-layer evaporation (TLE) technique [35-36]. Liposomes were then functionalized with a LOX antibody through conjugation to the carboxyl functional group via the PEG termini [37]. To obtain optimal membrane conjugation, three different anti-LOX-to-lipid ratios were tested: 1:1000, 1:500, and 1:300. An increase in anti-LOX ratios demonstrated a steady decrease in zeta potential, indicating successful incorporation into the bilayer (Figure 6A). In addition, physicochemical characterization of the three formulations demonstrated that anti-LOX coupling resulted in the shrinkage of the vesicular structure, as observed by a reduction in particle size when compared to control liposomes with no differences between the tested anti-LOX ratios (Figure 6B). Increased stabilization of the formulation was also discovered, as indicated by the decrease in the polydispersity index (Figure 6C). Next, flow cytometry analysis was performed to determine the optimal LOX-to-lipid ratio in terms of anti-LOX expression (Figure 6D). The analysis revealed that the 1:500 ratio expressed the highest intensity of LOX antibody compared to 1:300 and 1:1000. This result could depend on the saturation of the carboxyl functional group of liposomes with the use of 1:500 anti-LOX-to-lipid ratio, thus this antibody concentration was used for all of downstream experiments.

Anthracyclines and taxanes-based regimens represent one of the standard therapeutic option in TNBC patients. Although these molecules achieve higher pathological response rate (30.9-36.4%) [38] compared to other chemotherapy, they suffer from limited tumor targeting and systemic toxicity. For these reasons it is mandatory to find strategies to selectively concentrate anthracyclines to the tumor site. Specific ECM tumor-targeting and intrinsic antitumor activity of LOX could enhance the efficacy of standard anthracycline-based regimens by directing accumulation to tumors. For the above reason the inventors have designed an anti-LOX engineered drug delivery system to efficiently target and concentrate anthracyclines within the tumor ECM. Anti-LOX-functionalized liposomes (Lipo-LOX) were loaded with the chemotherapeutic, epirubicin, using previously established loading techniques [39]. This resulted in an encapsulation efficiency of 60% for both Lipo and Lipo-LOX, thus indicating no significant impact on drug loading from anti-LOX engineering (Figure 6E). In order to assess the efficacy of both anti-LOX and epirubicin, four different lipid-based formulations were synthesized: liposomes (Lipo), anti-LOX liposomes (Lipo-LOX), epirubicin-load liposomes (Lipo-EPI), and epirubicin-loaded anti-LOX liposomes (Lipo-EPI-LOX). The composition and the main physicochemical characteristics of the different preparations are reported in Figure 1A. Notably, the diameters of Lipo and Lipo-LOX were in the range of 156 to 171 nm, respectively, while increased sizes were observed following encapsulation of epirubicin (220 nm and above). These differences could be attributable to the drug loading as indicated also by the increased PDI polydispersity index value in Lipo-EPI and Lipo-EPI-LOX when compared to Lipo. In addition, the presence of anti-LOX antibody resulted in an increased surface charge (i.e., -23 to -21), likely depending from amine functional groups from the antibody structure. Conversely, incorporation of epirubicin resulted in a decrease in surface charge (i.e., -30 mV) suggesting successful entrapment of the drug. Validation of LOX antibody on the liposome surface was further confirmed, as previously showed in the Lipo-LOX designing study, using flow cytometry and also with Fourier Transform Infrared spectroscopy (FTIR) analysis, revealing successful incorporation of anti-LOX antibodies (Figures 1B and 1C).

### Example 2: Validation of the anti-LOX activity anchored to liposomes: in vitro evidences

The presence of functional LOX antibody on the liposome surface was confirmed by assessing the inhibition of secreted LOX protein in both standard monolayer (2D) and three dimensional (3D) cultures of MDA-MB-231 cells. LOX protein secretion by MDA-MB-231 cancer cells was not detectable in standard 2D culture both in control and treatment groups at various time points, confirming the need of ECM support for the production of LOX protein (Figure 1D). However, when MDA-MB-231 were cultured on a 3D collagen scaffold, LOX protein secretion was observed for 48h (Figure 1E). Secretion of LOX protein in 3D was likely possible as a response to the more aggressive features (lower oxigen tension, lower concentration of nutrients) of the 3D microenvironment and the contribution of collagen fibers. Moreover, these date are suggestive of the higher reliability of 3D culture systems in reproducing some of the key features of growing tumors. Thus in order to confirm that the synthesis process has not affected the anti-LOX activity when coupled to liposomes, the cultures were exposed to nanosystems and circulating LOX was analyzed. The results showed that LOX protein was detectable in Lipo, EPI, and Lipo-EPI, while its expression was initially inhibited in the presence of Lipo-EPI-LOX at 6h before expressing similar values to other groups (Figure 1E). These results suggest that liposomes functionalized with anti-LOX retain the bio-activity associated with anti-LOX, confirming the feasibility of implementing an anti-LOX-functionalized system for cancer therapy.

### Example 3: In vitro assessment of engineered-anti-LOX liposomes for tumor targeting, drug delivery and citotoxicity

Functional *in vitro* assays for investigating the tumor targeting and drug delivery ability of Lipo-EPI-LOX compared to standard treatment groups epirubicin and Lipo-EPI were performed. For these experiments MDA-MB-231 human TNBC cultured in 3D collagen-based systems were used. The analysis showed that both tumor targeting and drug delivery of the inventors' nanovesicle resulted significantly improved (EPI vs LIPO-EPI-LOX at 6h p=7.8 x 10⁻²¹ and LIPO-EPI vs LIPO-EPI-LOX at 6h *p*=6.1 x 10⁻¹⁷; EPI vs LIPO-EPI-LOX at 48h *p*=8.05 x 10⁻⁷ and LIPO-EPI vs LIPO-EPI-LOX at 48h *p*=1.4 x 10⁻⁵) when compared to both treatment groups at the same drug concentration over the time 6h and 48 h (Figure 2A-2C).

Next, the cytotoxic effect of Lipo-EPI-LOX on the viability of MDA-MB-231 human TNBC cells cultured in 2D and 3D models was assessed and compared to empty liposomes, Lipo-LOX, EPI, and Lipo-EPI (Figure 2D-2E). Specifically, in 2D, Lipo and Lipo-LOX had a slight effect on the viability. Lipo-EPI-LOX resulted in an 85% reduction in TNBC cell viability compared to control, with Lipo-EPI and EPI alone displaying similar values (Figure 2D). In 3D culture systems, Lipo-EPI-LOX showed a similar significant reduction in cell viability with >80% cell death observed. Meanwhile, Lipo and Lipo-LOX had no statistically significant effect on survival and Lipo-EPI and EPI reported <80% of cell death, respectively. These results confirmed that Lipo-EPI-LOX resulted in a similar effect in decreasing tumor cell proliferation compared to the other tested agents and that the incorporation of anti-LOX did not impact the drug features of EPI or liposomes. Interestingly, a higher activity of LIPO-EPI-LOX in 2D cultures was observed by reducing the drug concentration to as low as one quarter of the plasma peak of anthracyclines (Figure 7C and 7D) compared to plasma peak and half plasma peak (Figure 7A and 7B), suggesting that breast cancer cells reached drug saturation at both human plasma peak and half human plasma peak concentration.

The above consideration is closely linked to the 2D culture system where cells are in close contact with drug compounds making this model far from the human biology and partially causing the gap, still present between preclinical and clinical data. Furthermore, results from 2D (Figure 7A-7D) are not able to show the effective spectrum of anti-LOX activity, as previously demonstrated (Figure 1D). Results obtained in 3D cultures (Figure 8A-8D) showed an increase in cells viability with all of the tested formulations when the drug concentration was decreased to one quarter of the plasma peak (Figure 8C). Of note was that Lipo-EPI-LOX resulted in the higher inhibition of cell viability when compared to all treatment groups. These results may be explained by the fact that 3D collagen-based scaffold mimics better the in vivo growing tumors since it incorporates feature of the ECM. This mix of macromolecules influences tissue tension, oxygen, nutrients and drugs diffusion. For these reasons the *in vivo* tumor microenvironment is characterized by cells exposed to different drug contents. As a result, the development of drug delivery systems capable of concentrating drugs to tumor lesions provide new avenues for treatment of cancer, in particular TNBC. Finally, since the mechanical characteristics of tumors and tumor cells are predictive of the disease clinical behavior, the inventors investigated the effect of all treatment groups on the stiffness properties of collagen scaffolds. The results (Figure 10) showed an average compressive modulus of 67.3 kPa for CTR, 64.28 for Lipo, 61.78 for Lipo-LOX, 64.38 for free EPI, 59.81 for Lipo-EPI and 55.64 for Lipo-EPI-LOX. In these experiments only Lipo-EPI-LOX exhibited a significant reduction of scaffold stiffness (p<0.05) compared to CTR. These data support the importance of develop a drug delivery system able to modify the mechanical properties of tumor microenvironment as a new weapon for treatment of cancer, in particular TNBC.

### Example 4: In vivo biodistribution and tumor targeting of engineered-anti-LOX liposomes

A critical directive in drug delivery is instructing the selective delivery of toxic compounds to only targeted sites while avoiding healthy tissues. The biodistribution of EPI within Lipo-EPI and Lipo-EPI-LOX was evaluated using an orthotropic xenograft mouse model of human TNBC. Briefly, MDA-MB-231 were labeled with firefly luciferase and subcutaneously injected in Nu/Nu nude mice. When tumors reached a mean volume of 250-300 mm³ mice were intravenously administered with Lipo-EPI and Lipo-EPI-LOX. Biodistribution analysis in mice was assessed using BLI and fluorescence imaging to determine the distribution of tumor cells and EPI, respectfully. At 0, 1, 2, and 24 h mice were imaged and as early as 1h visible signals from EPI were observed in the tumors (outlined in red after confirmation of location with BLI) of Lipo-EPI-LOX mice and persisted till 24h (Figure 3A). In both groups, EPI signals in the liver (or abdomen) and thoracic area were seen but by 24h these signals had dissipated. At 24h, mice were sacrificed and their organs were collected and imaged again for EPI (Figure 3B). *Ex vivo* imaging of organs demonstrated that mice treated with Lipo-EPI-LOX exhibited more EPI accumulation in tumors and less accumulation in the liver compared to Lipo-EPI. Quantification of longitudinal imaging in Figure 3A confirmed that tumors treated with Lipo-EPI-LOX presented with significantly higher levels of EPI at 1 and 24h compared to Lipo-EPI (Figure 3C). Furthermore, quantification of EPI fluorescent signal in organs treated with Lipo-EPI-LOX revealed significantly higher accumulation of EPI in the tumors and significantly less accumulation in livers (Figure 3D). Taking together, this data suggests Lipo-EPI-LOX was successful in delivering more EPI to TNBC tumors than liposomal not decorated with anti-LOX.

### Example 5: Treatment with Lipo-EPI-LOX slowed the mammary tumor progression and prolonged the survival of MDA-MB-231 murine xenografts

In order to explore the therapeutic potential of Lipo-EPI-LOX the same TNBC tumor model described above was used. When tumors reached a mean volume of 250-300 mm³, mice were divided into groups and intravenously administered a weekly treatment of empty liposome (i.e., control (CTR)), Lipo-LOX, Lipo-EPI, Free-EPI and Lipo-EPI-LOX. Mice were treated for four weeks and sacrificed upon reaching a mean tumor volume of two cubic centimeters. Weekly tumor volume measurements revealed that treatment with Lipo-EPI-LOX yielded significant inhibition of TNBC growth compared to all treatment groups (Figure 4A). In particular, Lipo-EPI-LOX displayed a substantial reduction (810 mm³±174 mm³) in tumor growth compared to Lipo-EPI (1039 mm³±233 mm³) and free EPI (1029 mm³±203 mm³). Interestingly, Lipo-EPI and EPI were consistent with clinical observations where conventional anthracycline-based regimens and liposomal anthracycline exhibited equivalent antitumor efficacy [40]. Furthermore, the impact on tumor growth was validated by evaluating bioluminescent (BLI) signal of TNBC (Figure 4B and Figure 9). This was performed in order to confirm that data obtained with caliper measurement were linkable only to the presence of tumor cells exluding any off target effect. Interestingly, the BLI quantification percentage (D0 vs D33) was found to be statistically significant in CTR (*p*=0.01) and Lipo-LOX (*p*=0.01) groups, while free EPI (*p*=0.24), Lipo-EPI (*p*=0.09) and Lipo-EPI-LOX (*p*=0.35) did not result in statistical significance with the lowest signal detected with Lipo-EPI-LOX treatment group. These experiments demonstrated that mammary cancer progression can be slowed with anthracycline-based regimens, as observed into daily clinical practice, and that LIPO-EPI-LOX treatment provides superior antitumor efficacy among all treatment groups.

The decreased viability observed with BLI was further confirmed by hematoxylin and eosin (H&E) staining of tumor sections and analyzing for the percentage of necrotic cells. In particular necrotic tumor cells were 5% for control group, 25% in Lipo-LOX, 50% in Lipo-EPI, 20% for free EPI and 60% for Lipo-EPI-LOX (Figure 4C). These experiments confirmed the superior ability of Lipo-EPI-LOX in inducing tumor necrosis among all treatment groups. Moreover, since different works [41-42] have observed the progressive linearization of collagen fibers adjacent to tumor lesions which is linked by collagen crosslinking, ECM stiffening and increased focal adhesions we investigated the collagen of explanted tumors. The analysis showed a higher collagen linearization in CTR, LIPO-EPI and LIPO-EPI while a decrease in collagen stiffness and linearization was observed with LIPO-LOX and LIPO-EPI-LOX. Reducing lysyl oxidase-mediated collagen crosslinking decreased focal adhesions and PI3K activity, impeded malignancy and lowered tumor incidence. Thus, these results demonstrate the ability of Lipo-EPI-LOX in reducing tumor progression among the tested treatment groups.

Following the establishment of Lipo-EPI-LOX antitumor activity, the inventors next investigated the overall survival between treatment conditions (Figure 4D). Specifically, following the conclusion of the survival study (i.e., 55 days post treatment), the inventors observed a >75% survival for Lipo-EPI-LOX whereas control and free-EPI experienced survival rates less than 50% and less than 65% for Lipo-LOX and Lipo-EPI. As such, Lipo-EPI-LOX resulted in a significant increase in survival (p=0.032) when compared to control and tested treatment groups. In addition, while Lipo-LOX prolonged survival when compared to an anthracycline regimen, but not to Lipo-EPI confirming the potential synergistic effect mediated by both LOX and EPI when administered in the same formulation. These findings provide a proof-of-concept assessment of Lipo-EPI-LOX as a viable therapeutic option for cancer patients, in particular TNBC.

### Example 6: Safety of Lipo-EPI-LOX treatment on MDA-MB-231 murine xenografts

Since a major limitation of chemotherapy is represented by systemic toxicity the inventors analyzed the safety profile of the inventors' drug delivery system in *in vivo.* In particular, the inventors evaluated the safety of LIPO-EPI-LOX by assessing the total change in body weight (Figure 5A) and cardiotoxicity (Figure 5B). Abnormal alterations in body weight can be used as a surrogate of the overall health status and tolerability of systemically administered substances [43]. Changes in body weight were calculated at the end of the study [(D33^{∗}100)/D0], and resulted in the following increases: 3.3% in CTR (p=0.05), 2.9% in Lipo-LOX (p=0.008), 2.9% in Lipo-EPI (p=0.06), 3.7% in free EPI (p=0.10) and 6.0% in Lipo-EPI-LOX (p=0.004). These results suggested the higher tollerability of Lipo-EPI-LOX treatment, which is directly correlated to the lower fatigue and increased appetite.

Anthracycline-based regimens have a well-documented history of cardiotoxicity. For this reason, we evaluated the impact of our therapy on the architecture of the heart. Analysis on H&E hearts confirmed the safety of Lipo-EPI-LOX in preventing organ-related toxicity (Figure 5B). Myocardial tissues treated with Free EPI and Lipo-EPI showed an increment of cellularity due to the inflammatory infiltrate (granulocytes and lymphocytes) (Fig. 5B). Conversely, myocardial tissues from Lipo-LOX and Lipo-EPI-LOX groups did not show a significant increase in the inflammatory infiltrate. These results indicate that Lipo-EPI-LOX did not trigger a significant adaptive immune response in hearts against lipid-vesicles and suggests its ability to avoid significant cardiotoxicity *in vivo.* These results indicate Lipo-EPI-LOX treatment group resulted in better overall performance when compared to the other treatment groups.

In summary, the inventors demonstrated the efficacy of LOX-engineered lipid vesicles loaded with epirubicin for the treatment of triple negative breast cancer. In this context, the inventors showed the combination of a LOX antibody and an anthracycline into a lipid-based nanocarrier was not only feasible but also successful in providing significant advantages in terms of therapeutic activity and reduced toxicity. Lipo-EPI-LOX was assembled through the simple and well-established TLE method and further functionalized with the LOX antibody. *In vitro* experiments indicated superior tumor targeting and cytotoxic activity of Lipo-EPI-LOX compared to all the tested formulations. In addition, *in vivo* experiments revealed Lipo-EPI-LOX maintained higher therapeutic activity, with an increase in survival percentage. When compared to current clinical standards, Lipo-EPI-LOX further demonstrated a reduction in systemic toxicity, solidifying its potentials as a viable therapeutic strategy for breast cancer and as an ECM-targeting formulation.

### REFERENCES

[1] Janmey, P. A., and R. T. Miller. J. Cell Sci. 124:9-18, 2011.
[2] Levental, K. R. et al. Cell 139:891-906, 2009.
[3] Psaila, B., and D. Lyden. Nat. Rev Cancer 9:285-293, 2009.
[4] Chiara Liverani, et al. Cel. Mol. Bioeng. (2017) 10: 223.
[5] Rodriguez HM, et al. J Biol Chem. 2010 Jul 2;285(27):20964-74.
[6] Barry-Hamilton V, et al. Nat Med. 2010 Sep;16(9):1009-17.
[7] Barker HE, et al. Cancer Res. 2011 Mar 1;71(5):1561-72.
[8] Miller BW, et al. EMBO Mol Med. 2015 Aug;7(8):1063-76.
[9] Erler JT, et al. Cancer Cell. 2009 Jan 6;15(1):35-44. doi: 10.1016/j.ccr.2008.11.012.
[10] Erler JT, et al. Nature. 2006 Apr 27;440(7088):1222-6.
[11] Levental KR, et al. Cell. 2009 Nov 25;139(5):891-906.
[12] Cox TR, et al. Nature. 2015 Jun 4;522(7554):106-110.
[13] Taylor MA, et al. Neoplasia. 2011 May;13(5):406-18.
[14] Payne SL, et al. J Cell Biochem. 2007 Aug 15;101(6):1338-54.
[15] Kagan HM, et al. J Cell Biochem. 2003 Mar 1;88(4):660-72.
[16] Butcher DT, et al. Nat Rev Cancer. 2009 Feb;9(2):108-22.
[17] Li W, et al. J Cell Biochem. 2000 Jun 12;78(4):550-7.
[18] Giampuzzi M, et al.Biochim Biophys Acta. 2003 Apr 11;1647(1-2):239-44.
[19] Bais MV, et al. PLoS One. 2012;7(2):e31188.
[20] Chang J, et al. Oncotarget. 2017 Apr 18;8(16):26066-26078.
[21] Palamakumbura AH, et al. Oncogene. 2009 Sep 24;28(38):3390-400.
[22] Rachman-Tzemah C, et al. Cell Rep. 2017 Apr 25;19(4):774-784.
[23] Johnston KA, et al. Cancer Lett. 2018 Mar 28;417:174-181.
[24] Philip C. Trackman. Lysyl Oxidase Isoforms and Potential Therapeutic Opportunities for Fibrosis and Cancer. Expert Opin Ther Targets. Author manuscript; available in PMC 2017 Aug 1. Published in final edited form as: Expert Opin Ther Targets. 2016 Aug; 20(8): 935-945. Published online 2016 Mar 3.
[25] Granchi C, et al. ChemMedChem. 2009 Oct;4(10):1590-4.
[26] Kanapathipillai M, et al. Nano Lett. 2012 Jun 13;12(6):3213-7.
[27] De Vita A, et al. Ther Adv Med Oncol. 2017 Dec;9(12):755-767.
[28] Fogli S, et al. Ann Oncol. 2002 Jun;13(6):919-27.
[29] Freireich EJ, et al. Cancer Chemother Rep. 1966 May;50(4):219-44.
[30] Näkki S, et al. ACS Appl Mater Interfaces. 2017 Jul 19;9(28):23441-23449.
[31] The effect of multistage nanovector targeting of VEGFR2 positive tumor endothelia on cell adhesion and local payload accumulation. Martinez JO, Evangelopoulos M, Karun V, Shegog E, Wang JA, Boada C, Liu X, Ferrari M, Tasciotti E.
[32] Short and long term, in vitro and in vivo correlations of cellular and tissue responses to mesoporous silicon nanovectors. Martinez JO, Boada C, Yazdi IK, Evangelopoulos M, Brown BS, Liu X, Ferrari M, Tasciotti E. Small. 2013 May 27;9(9-10):1722-33.
[33] Chiara Liverani, et al. Cel. Mol. Bioeng. (2017) 10: 223.
[34] Barenholz Y. J Control Release. 2012 Jun 10;160(2):117-34.
[35] A. Laouini, et al. Journal of Colloid Science and Biotechnology 1, 147-168 (2012).
[36] Molinaro R, et al. Nat Mater. 2016 Sep;15(9):1037-46
[37] Gao J, et al. Biomaterials. 2012 Jan;33(1):270-82.
[38] Cortazar P, et al. Lancet. 2014 Jul 12;384(9938):164-72.
[39] Kirui DK, et al. Adv Healthc Mater. 2015 May;4(7):1092-103.
[40] Harris L, et al. Cancer. 2002 Jan 1;94(1):25-36.
[41] Levental KR, et al. Cell. 2009 Nov 25;139(5):891-906. doi: 10.1016/j.cell.2009.10.027.
[42] Shanbhag V, et al. Proc Natl Acad Sci U S A. 2019 Apr 2;116(14):6836-6841. doi: 10.1073/pnas.1817473116. Epub 2019 Mar 19.
[43] Ullman-Cullere MH, et al. Lab Anim Sci. 1999 Jun;49(3):319-23.

## Claims

1. A liposome comprising:
- a poly(ethylene glycol)-lipid (PEG-lipid) or a derivative thereof;
- cholesterol
- an anti-LOX antibody and
- a therapeutic agent.

2. The liposomal according to claim 1, wherein the PEG-lipid is selected from the group consisting of: 1,2-distearoyl-sn-glycero-3-phosphocholine (DSPC), diacyl-phosphatidylethanolamine-N-[methoxy(polyethene glycol)]; distearoyl-phosphatidylethanolamine-N-[methoxy(polyethylene glycol)-2000] (DSPE-PEG-2000); and distearoyl-phosphatidylethanolamine-N-[methoxy(polyethylene glycol)-5000] (DSPE-PEG- 5000).

3. The liposome according to claim 1 wherein the anti-LOX antibody is functionalized through conjugation to the carbonyl functional group via the PEG termini.

4. The liposome according to claim 1 or 2 wherein the therapeutic agent is a chemotherapy, preferably said chemotherapy is selected from the group consisting of: a chemotherapeutic agent, an immunotherapeutic agent, a targeted therapy and a radiometabolic agent alone or in combination.

5. The liposome according to any one of previous claim wherein said liposome has a Z average from 150 to 250 nm or a Pdi from 0.02 to 0.2 or a Z Potential from -20 to - 30 mV or a EE% from 40 to 60%.

6. The liposome according to any one of previous claim wherein the anti-LOX antibody:PEG-lipid molar ratio is from 1:100 to 1:5000, preferably from 1:300 to 1:1000, preferably 1:500.

7. A formulation comprising the liposome according to any one of previous claim and a pharmaceutically-acceptable excipient.

8. The liposome according to any one of claim 1 to 6 or the formulation according to claim 7 for medical use, preferably for use in the treatment of cancer.

9. The liposome or the liposomal formulation for use according to claim 8 wherein the cancer is a solid tumor or a hematological malignancy, preferably the tumor is a triple negative breast tumor.

10. A method of production of a liposome according to any one of claim 1 to 6 comprising the steps of:
- combining a poly(ethylene glycol)-lipid (PEG-lipid) or a derivative thereof with cholesterol to obtain a lipid suspension;
- adding an anti-LOX antibody to said lipid suspension and
- adding a therapeutic agent.
